Europäisches Patentamt

European Patent Office

Office européen des brevets

(19)

(11) Numéro de publication: **0 510 167 B1**

(12) **FASCICULE DE BREVET EUROPEEN**

(45) Date de publication du fascicule du brevet: **23.08.95**

(51) Int. Cl.⁶: **C07J 1/00**, A61K 31/56, C07J 5/00, C07J 7/00, C07J 11/00

(21) Numéro de dépôt: **92900237.6**

(22) Date de dépôt: **12.11.91**

(86) Numéro de dépôt internationale : **PCT/FR91/00888**

(87) Numéro de publication internationale : **WO 92/08730 (29.05.92 92/12)**

(54) **NOUVEAU PROCEDE DE CRISTALLISATION DE SUBSTANCES ORGANIOUES D'ORIGINE STEROIDIENNE ET LES COMPOSES AINSI OBTENUS.**

(30) Priorité: **12.11.90 FR 9013981**

(43) Date de publication de la demande: **28.10.92 Bulletin 92/44**

(45) Mention de la délivrance du brevet: **23.08.95 Bulletin 95/34**

(84) Etats contractants désignés: **AT BE CH DE DK ES FR GB GR IT LI LU NL SE**

(56) Documents cités:
| | |
|---|---|
| DE-A- 2 323 812 | FR-A- 2 271 833 |
| US-A- 2 096 744 | US-A- 2 361 847 |
| US-A- 2 897 216 | US-A- 3 007 923 |
| US-A- 3 053 865 | |

CHEMICAL ABSTRACTS, vol. 103, no. 14, 7 Octobre 1985, Columbus, Ohio, US; abstract no. 106156, A. MATYNIA: 'Surface Active Agents in Regeneration of a Spinning Bath' voir abrégé & WLOKNA CHEM. vol. 10, no. 3, 1984, pages 257 - 263;

JOURNAL OF THE AMERICAN CHEMICAL SOCIETY. vol. 79, no. 16, 23 Août 1957, GASTON, PA US pages 4472 - 4475; D. GOULD ET AL: 'Long Acting Testosterone Esters. Some Considerations on their Biological Utilization' voir le document en entier

(73) Titulaire: **LABORATOIRE THERAMEX S.A. Immeuble des Industries, 6, Avenue du Prince Héréditaire Albert MC-98000 Monaco (MC)**

(72) Inventeur: **LANOUETIN, Michel Ouartie l'Adrech, Laghet F-06340 La Trinité (FR)**

(74) Mandataire: **Burtin, Jean-François Cabinet GEFIB 85 Rue Anatole France F-92300 Levallois Perret (FR)**

EP 0 510 167 B1

## Description

La présente invention se rapporte au domaine de la pharmacotechnique et plus particulièrement à un procdé d'obtention de principes actifs à usage pharmaceutique, de granulométrie contrôlée par une méthode de recristalisation.

Il est connu, en effet, que la taille de cristal d'un principe actif pharmaceutique joue un rôle important lors de la réalisation de formes pharmaceutiques sèches ou liquides pour assurer une réalisation reproductible de la forme, et ensuite une résorption constante.

On cite couramment dans la littérature que des variations de cinétiques de dissolution sont dues aux modifications de la structure cristalline ou des propriétés de surface des cristaux ou aux modifications de l'importance de la surface de contact mise en jeu [G.GILLARD Labo. Pharm. - Prob. et Tech. n° 309, 359 à 369 (1981)]

Pour améliorer la cinétique de dissolution des principes actifs de médicaments présentant une solubilité limitée, on utilise généralement la diminution de la taille des particules.

On résout ce problème habituellement par l'emploi d'une méthode mécanique telle que le broyage ou une micronisation (broyage par jet d'air).

Une étude menée sur différents progestatifs par MUTTENRAUCH et Coll. [STP Pharma 5(10), 642-646 (989)] a montré que l'effet de la taille des particules sur la vitesse de dissolution était étroitement lié à la solubilité des substances.

On a souvent décrit sur un grand nombre de molécules à emploi thérapeutique que la taille des particules et les propriétés physicochimiques des principes actifs qui résultent de ces traitements, conditionnent la biodisponibilité des formes pharmaceutiques en contenant, par l'intermédiaire des modifications des taux et vitesses de dissolution [cf. FDA paper-Guidelines Manuf Control Form ANDAS. 1985].

Toutefois, ces méthodes de broyage ne conviennent pas pour pulvériser tous les principes actifs dont certains sont de bas points de fusion et deviennent pâteux ou élastiques.

D'autres principes actifs à cause de leurs caractères physiques ne peuvent pas être micronisés (80% < 10 $\mu$m) et cela malgré plusieurs passages au broyeur.

Il a été décrit, notamment par NAKAGAWA et Coll. [Chem. Pharm. Bull 30, 242 (1982)] que la surface spécifique et la cristallinité ont une très grande influence sur la stabilité chimique à l'état solide du produit pulvérulent.

La vitesse de dissolution également est tributaire de la cristallinité du produit ; comme B.A HENDRIKEN l'a décrit dans Int. Journ. of Pharm. 60, 243-252 (1990).

D'autres investigateurs ont étudié les effets des traitements mécaniques, comme le broyage, sur les propriétés physico-chimiques de nombreux principes actifs entrant dans la composition d'une forme galénique à usage thérapeutique.

Les auteurs ont noté que les principes actifs avaient leurs caractéristiques physico-chimiques modifiées par ces traitements.

Parmi celles-ci, on peut citer :
- la perte de cristallinité (vérifiée par diffraction aux rayons X)
- la variation de leur surface spécifique SW (qui peut doubler ou tripler)
- une chute de la stabilité chimique vérifiée par analyse thermique différentielle de la température de décomposition (pour certains principes actifs, on a relevé des chutes de points de fusion de 10 à 15°)
- une variation de leurs propriétés de surface, ce qui ne facilite pas toujours la fabrication d'un mélange de poudres. Il a été décrit plus spécialement, dans l'article de GILLARD précédemment cité, que les propriétés morphométriques, électriques et rhéologiques étaient très importantes pour la réalisation d'un mélange homogène et de bon pouvoir d'écoulement.

Tous ces changements des caractères physico-chimiques sur différents principes actifs ont été très bien décrits par M.OTSUKA et N.KANENIWA dans l'International Journal of Pharmaceutics 62, 65 à 73 (1990) et les références citées dans cet article.

En outre, on ne peut pas oublier l'influence de la réduction de la taille des particules obtenues par broyage sur la dureté des comprimés, obtenus à partir de ces produits [cf. SAGAWA Y. - J. Powder Technol. Jap. 20, 737-743 (1983).

C'est pourquoi les résultats expérimentaux de TAWASHI STP Pharma. 6(5), 299-302 (1990) ont fourni une illustration de la relation qui existe entre la réduction de taille des particules et l'aspect morphologique des fragments en résultant. L'effet du mécanisme de réduction selon les moyens utilisés sur les dimensions des fragments concerne essentiellement la mise en évidence de l'irrégularité des surfaces des particules et la correlation qui existe entre les surfaces de particules avec le comportement physique des poudres lors de la production de formes pharmaceutiques. L'aspect rugueux de la surface par son influence sur l'aptitude à l'écoulement du produit constitue un des facteurs importants qui ont une influence sur la qualité d'un mélange de poudres, en vue de la réalisation d'une telle forme pharmaceutique. L'effet du mécanisme de réduction joue encore un rôle important sur la solubilité du principe actif.

D'autres études ont également montré que la forme cristalline d'un principe actif pouvait encore subir une transformation (recristallisation) ou une déformation (plastique) en cours de compression [cf. C.FUHRER, STP PHARMA 6(5), 294-298 (1990)].

Le broyage peut également avoir pour effet de remplacer les cristaux par des agglomérats équivalents de cristaux plus petits, qui n'améliorent en aucune façon la solubilité, ni la vitesse de dissolution du principe actif.

De plus, un broyage, s'il est mécanique comme c'est le cas avec des broyeurs à couteaux, peut introduire des souillures du produit par des particules métalliques ou par de l'huile.

L'objet de l'invention est donc de trouver une solution plus satisfaisante que le broyage, pour obtenir une classe granulométrique de cristaux, utilisables dans l'industrie du médicament, en obtenant à volonté de cristaux d'une taille déterminée, en pourcentage quasiment constant, et qui évite d'utiliser des méthodes traumatisantes de réduction de particules comme les diverses publications en ont fait mention et que la demanderesse a pu elle-même le constater sur les principes actifs.

Il s'est donc avéré possible d'arriver à une solution de ce problème physique de cristallisation et d'obtenir ainsi une classe granulométrique homogène de cristaux, sans qu'il soit nécessaire d'avoir recours à un broyage ni à un tamisage et sans même modifier le système cristallin du produit de départ. Ceci est vérifié par microscopie et analyse thermique différentielle.

La demanderesse a donc cherché, pour éviter l'utilisation d'une méthode de broyage, à exercer une influence sur les divers facteurs qui régissent les phénomènes de la cristallisation et notamment à modifier les constituants de la masse à cristalliser. Ces procédures ont pour but d'exercer une influence principalement sur des facteurs déterminants comme les transferts de masse et de chaleur. On sait, en effet, que le transfert de masse est dû à un phénomène de diffusion des molécules de la masse liquide vers la surface des cristaux et que le transfert de chaleur est provoqué par la diffusion de la chaleur de cristallisation (énergie de liaison relâchée durant la période de formation du cristal) de la surface des cristaux vers la masse du liquide. Une maitrise suffisante de la superposition de ces deux transferts a permis d'exercer une influence sur la croissance des cristaux et ainsi, d'éviter le facteur de proximité des cristaux lors de leur formation, en solution concentrée. Ce dernier facteur intervient pour perturber la croissance régulière des cristaux en provoquant la formation d'agglomérats.

L'invention a donc précisément pour objet un procédé de cristallisation qui, par cette influence sur les facteurs de la cristallisation, permet sans opération mécanique d'obtenir une classe granulométrique homogène que l'on peut déterminer à l'avance avec précision, caractérisé en ce que le produit que l'on recherche à faire cristalliser est au préalable dissout dans un mélange ternaire composé d'un solvant lipophile, d'un solvant hydrophile et d'un agent tensio-actif, à une température proche de l'ébullition du mélange de solvants et qu'on refroidit cette solution à une température où la cristallisation s'amorce, puis sépare les cristaux formés qui sont de même système cristallin que ceux initialement utilisés avant la dissolution dans les solvants, mais de la taille recherchée.

Des procédés similaires, mais en mélange binaire, ont déjà été décrits pour la recristallisation de sels minéraux, comme par exemple CHIANESE A. et Coll, Process Technol Proc. 89, Vol.6 (ind cryst 87), 261-264 sur le perborate de sodium qui utilisent un surfactant dans l'eau pour recristalliser ce sel ou comme BLASZCZAK J. et coll., Kryst Przem. Krajowe Symp, Mater Konf, 3rd (56 WYAM) 89, 95-101, sur le fluorure d'aluminium hydraté.

Dans la littérature, on trouve également des publications qui étudient la cristallisation pour obtenir des principes actifs dans un système cristallin bien défini, comme par exemple SAS, G.A.J.M.T et coll. qui utilisent un procédé par précipitation pour obtenir le principe actif cristallisé en système monoclinique (Eur. Pat. Appl. 389.035 - CA (1991) 114:214521S), ou alors pour séparer des formes polymorphiques comme la Terfénadine (2 formes cristallines) (cf. T.G FAWCETT et coll, US Patent n° 4.742.175). D'autres auteurs préparent des microcristaux par précipitation dans un "anti-solvant". SCHMITT W. réalise ce procédé de microcristallisation en dissolvant le principe actif dans un solvant hydrosoluble et par injection dans le $CO_2$, et obtient des microcristaux (cf. PLT Int. Appl. WO 90.03.782 - CA (1990) 113:178284K).

Dans le procédé de l'invention, le solvant hydrophile est choisi de façon à être miscible ou à se dissoudre dans le solvant lipophile pour assurer une solution homogène. Il est de préférence formé d'un mélange aqueux et en particulier de l'eau additionnée ou non d'un solvant polaire et/ou d'ester d'alcoyles inférieur.

Après de nombreux essais, il s'avère avantageux de choisir un mélange ternaire qui est composé de solvants choisis parmi la famille des alcools (conme le méthanol, l'éthanol, le butanol, l'isopropanol), des cétones (comme l'acétone, la méthyléthylcétone ou la méthylisobutylcétone), l'acétate d'éthyle, l'isobutylacétate entre autres et de l'eau dont le pourcentage dans le mélange binaire est compris entre 0 et 12%.

L'agent tensio-actif est de préférence un agent tensio-actif non ionique choisi parmi les esters polyoxyéthyléniques de sorbitane et d'acide gras ayant au moins 8 atomes de carbone, les éthers polyoxyéthyléniques d'alcool gras ayant au moins 8 atomes de carbone et, les esters polyoxyéthyléniques d'acide stéarique.

L'agent tensio-actif non ionique est choisi parmi ceux qui sont de caractère amphiphile, mais à prédominance hydrophile, d'un HLB > 12 comme par exemple les esters polyoxyéthyléniques de sorbitane et d'acide gras comme les TWEEN 20 à 40, les éthers polyoxyéthyléniques d'alcool gras comme les BRIJ 56-58-78-96-97-98-99, G3816 et 3820, G3910 et 3920 ou ETHYLAN D254 à 257 ou RENEX ou CREMOPHOR ou encore type PLURONIC F68.

Les esters polyoxyéthyléniques d'acide stéarique comme les MYRJ 49, 51, 52, 53, 59 conviennent également car ils améliorent également la dissolution des principes actifs, permettent d'avoir des solutions très concentrées lors de la cristallisation et permettent d'abaisser également la température de recristallisation pour sursaturation du milieu.

En fonction des familles de principes actifs utilisés, il est préférable d'utiliser un mélange ternaire composé de solvants précités contenant 1 à 12% d'eau et de 0,01 à 10% d'agent surfactant et d'une manière tout à fait préférée de 0,05 à 5%.

Ce mélange ternaire peut être préparé en une seule opération ou bien en plusieurs opérations successives en dissolvant d'abord le principe actif dans un des solvants en additionnant éventuellement l'agent tensio-actif puis en complétant avec l'autre solvant.

Le volume et la nature du mélange ternaire à utiliser pour la recristallisation seront choisis en fonction de la classe granulométrique recherchée pour la forme pharmaceutique à étudier et en fonction du rendement prévisible.

Pour un principe actif défini, il est nécessaire d'établir un diagramme ternaire du mélange à utiliser et de définir les proportions des solvants le composant. Le volume du mélange ternaire est tributaire de la solubilité du principe actif au reflux dans ce mélange. Une bonne solubilité est nécessaire pour avoir des concentrations assez élevées dans la masse, car celle-ci est un facteur influent de la croissance des cristaux au cours du refroidissement.

La présence d'eau est nécessaire pour certains surfactants pour assurer une bonne répartition de ceux-ci dans la masse liquide et pour ainsi favoriser son incorporation dans le réseau, former des masses liquides et solides au cours de la recristallisition et influencer ainsi la tension interfaciale liquide/solide. L'effet de proximité en cours de cristallisation peut perturber la croissance régulière des cristaux et conduire à des groupements n'ayant pas une forme bien nette. C'est uniquement la disposition ordonnée dans l'espace des particules qui le composent qui distingue les cristaux, des substances amorphes où la disposition des particules est anarchique.

La température de chauffage du mélange ternaire joue un rôle important. Le mélange ternaire contenant le principe actif a recristalliser est amené à une température la plus proche possible de l'ébullition dudit mélange de manière à assurer une concentration importante de la substance à recristalliser et de manière à pouvoir abaisser, le plus possible, la température de recristallisation de la substance.

La taille des cristaux varie également en fonction des concentrations et des rapports solvant lipophile/solvant hydrophile. La quantité de solvant hydrophile passe par une valeur optimale, comme par exemple, avec les substances progestatives. Elle se situe entre 2 et 5% en présence d'un solvant de type cétonique, comme la méthyléthylcétone, et ceci pour obtenir une classe granulométrique de 35 à 70 $\mu$m, et préférentiellement à 5% pour resserrer cette classe entre 35 à 55 $\mu$m.

A 7.5 % avec le même solvant précité, la classe granulométrique remonte entre 70 et 100 $\mu$m. L'augmentation de la teneur en solvant hydrophile conduit à des cristaux de taille plus élevée. En outre, le fait d'opérer en milieu plus ou moins dilué, conduit également à des cristaux de taille plus ou moins grande et tributaire du surfactant utilisé. La règle selon laquelle on obtient des cristaux plus petits lorsque le principe actif est en concentration importante dans son milieu de cristallisation en monosolvant, n'est pas toujours vérifiée dans le présent procédé de recristallisation.

La vitesse de refroidissement, la température de début de recristallisation, la nature du mélange ternaire et la concentration en principe actif varient en fonction de la classe granulométrique recherchée et l'apport de frigories, ainsi que l'agitation du milieu seront définis à l'avance pour chaque essai. L'isolement du principe actif par filtration s'effectuera à des températures qui pourront s'échelonner entre +45° et -10°.

La transposition industrielle de ce procédé pour certaines substances a donné lieu à quelques décalages dans la classe granulométrique, c'est ainsi que pour le Promestriène qui sur 1 g donnait des cristaux d'environ 50 $\mu$m, a fourni sur un essai de 60 kg une granulométrie de 150 $\mu$m. Une mise au point sera nécessaire lors de la transposition industrielle.

Le principe actif est de préférence un composé à structure stéroïdienne et en particulier un dérivé de l'estrane, de l'androstane, du pregnane, du 19-nor pregnane ou du cholestane.

Parmi les dérivés de l'estrane, on pourra citer l'estradiol, l'estrone, l'estriol ou la nor-testostérone, ainsi que leurs éthers et/ou leurs esters. Un exemple plus particulier de dérivé de l'estrane est l'éther propylique du 17-méthyl éther de l'estradiol (Promestriène) ou l'undécanoate de 19-nor testostérone.

Parmi les dérivés de l'androstane, on pourra citer la testostérone, ses ethers et esters en 17, les testostérones substituées en position 4,6,7 ou 16, comme par exemple, la 4-chorotestostérone, la 6-méthyltestostérone, la 7-méthyltestostérone, les esters d'acide gras de la testostérone, comme le cyclopentyl acétate ou le cyclohexyl propionate de testostérone. Les dérivés 17 substitués d'androstène-2, comme le 17 $\beta$-acétoxy-17$\alpha$-éthynyl-5$\alpha$-androstène-2.

Parmi les dérivés du pregnane, on pourra citer la progestérone, ses éthers énoliques, les énamines cycliques ou linéaires, les dérivés 17$\alpha$-hydroxylés, les éthers de 17$\alpha$-hydroxyprogestérone, les esters de 17$\alpha$-hydroxyprogestérone, les progestérones substituées en position 1, en position 6, en position 7 ou en position 16.

Parmi les dérivés 21-hydroxy-pregnène, on pourra citer les molécules cortisoniques comme la cortisone, le cortisol, la prednisone, le médrol, la dexaméthasone, la bétaméthasone ou la triamcinolone.

Parmi les dérivés de la 19-nor progestérone, on pourra citer la 17$\alpha$-hydroxy 19-nor progestérone, ses éthers en 17, ses esters en 17, ainsi que les 19-nor progestérone substituées comme la 6-méthyl 17$\alpha$-hydroxy 19-nor progestérone, ses éthers en 17 et ses esters en 17, ainsi que le 6-méthy1-3,20-dioxo-17$\alpha$-hydroxy-19-nor-4,6-diène et ses esters.

Parmi les dérivés du cholestane, on pourra citer les acides biliaires, le cholestérol et ses esters, l'ergostérol, le stigmastérol, le calciférol.

Les composés micro-cristallins obtenus selon le procédé de l'invention servent de principes actifs dans des compositions pharmaceutiques sèches comme des comprimés ordinaires ou à libération modifiée, des gélules ou des granulés ; ou dans des compositions pharmaceutiques liquides comme des suspensions buvables ou des suspensions injectables pour l'administration intra-musculaire ou intra-articulaire dans des préparations vaginales, comme des suspensions dans des gels bio-adhésifs, des suppositoires ou des ovules.

Les exemples suivants illustrent l'invention sans toutefois la limiter. Ils montrent l'intérêt du présent procédé par rapport au broyage.

## EXEMPLE I

## MICROCRISTAUX D'HYDROCORTISONE

Dissoudre au reflux 10 g d'hydrocortisone dans 10 volumes d'un solvant formé de :
- 95,8 % de méthyléthylcétone
- 4,0 % d'eau
- 0,2 % de Tween 20

On maintient le reflux 5 à 10 minutes sous agitation et on s'assure qu'il ne reste plus de particules en suspension. Sous agitation, on refroidit la masse à -10°. On maintient cette température 1 heure et essore les cristaux formés. On lave les cristaux à l'eau et on les sèche sous pression réduite à une température voisine de 50/60°. Les cristaux ainsi formés vont de 25 x 30 $\mu$m pour les plus gros à 5 x 10 $\mu$m pour les plus petits.

$PF_k$ : 221,5-222°

$\alpha_D$ (méthanol) + 156 ± 2°

## EXEMPLE II

## MICROCRISTAUX DE DEXAMETHASONE ACETATE

Dissoudre au reflux 10 g d'acétate de Dexaméthasone dans 4 volumes d'un solvant formé de :
- 89,0 % de méthyléthylcétone
- 10,5 % d'eau

- 0,5 % de Mirj 51

On maintient le reflux pendant 30 minutes. On filtre les particules insolubles et on refroidit le filtrat à -15°. On maintient cette température 1 heure avant d'essorer la masse cristalline. On lave les cristaux à l'eau et les sèche à 60° sous pression réduite. La taille des cristaux ainsi formés va de 80 x 50 $\mu$m pour les plus gros, à 8 x 10 $\mu$m pour les plus petits.

PF$_K$ : 226-227°

$\alpha_D$ (Methanol) + 84 ± 2°

**EXEMPLE III**

**MICROCRISTAUX D'ACETATE DE DEXAMETHASONE**

On dissout au reflux 10 g d'acétate de Dexaméthasone dans 4 volumes d'un solvant formé de :
- 87,6 % d'acétone
- 12,0 % d'eau
- 0,4 % de Tween 20

On isole comme à l'exemple précédent et on obtient des cristaux qui vont pour les plus gros de 200 x 75 $\mu$m et pour les plus petits de 100 x 50 $\mu$m.

PF$_K$ : 228°

$\alpha_D$ (Méthanol) + 86 ± 2°

**EXEMPLE IV**

**MICROCRISTAUX DE PREDNISONE**

On dissout au reflux 10 g de Prednisone dans 5 volumes d'un solvant formé de :
- 94,8 % de méthyléthylcétone
- 5,0 % d'eau
- 0,2 % de Tween 20

On isole comme il est décrit dans les essais précédents (refroidissement -10°). On obtient des cristaux dont la taille maximum est d'environ 55 x 40 $\mu$m et la taille minimum 35 x 24 $\mu$m.

PF$_K$ : 240° ± 1°

$\alpha_D$ (Méthanol) + 167° ± 4°

**EXEMPLE V**

**MICROCRISTAUX D'ACETATE DE NOMEGESTROL**

On dissout au reflux 10 g d'acétate de Nomegestrol dans 6 volumes d'un solvant formé de :
- 94,9 % de Méthanol
- 5,0 % d'eau
- 0,1 % de Tween 20

On laisse revenir très lentement la température sous agitation par refroidissement externe jusqu'à -5°C. On maintient à cette température pendant 15 mn. On sépare la masse cristalline, on l'essore puis on lave à l'eau et on fait sécher sous pression réduite.

Les cristaux ainsi formés vont de 50 x 25 $\mu$m pour les plus gros à 10 x 10 $\mu$m pour les plus petits.

**EXEMPLE VI**

**MICROCRISTAUX D'ACETATE DE NOMEGESTROL**

On opère comme à l'exemple I, mais on utilise 2 volumes d'un solvant ternaire formé de :
- 92,4 % de Méthyléthylcétone
- 7,5 % d'eau
- 0,1% % de Tween 20

On recueille des microcristaux dont la taille s'échelonne de 100 x 100 $\mu$m pour les plus gros à 65 x 35 $\mu$m pour les plus petits cristaux.

### EXEMPLE VII

**MICROCRISTAUX D'ACETATE DE NOMEGESTROL**

On opère comme à l'exemple I, mais on utilise 2 volumes d'un solvant ternaire formé de :
- 94,9 % de méthyléthylcétone
- 5,0 % d'eau
- 0,1 % de Tween 20

On recueille des microcristaux dont la taille s'échelonne de 55 x 40 $\mu$m pour les plus gros à 35 x 25 $\mu$m pour les plus petits.

### EXEMPLE VIII

**MICROCRISTAUX D'ACETATE DE NOMEGESTROL**

On opère comme à l'exemple I, mais on utilise 2 volumes d'un solvant ternaire formé de :
- 87,9 % de Méthyléthylcétone
- 12,0 % d'eau
- 0,1 % de Tween 20

On recueille des microcristaux dont la taille s'échelonne de 150 x 65 $\mu$m pour les plus gros à 90 x 50 $\mu$m pour les plus petits.

### EXEMPLE IX

**MICROCRISTAUX D'ACETATE DE NOMEGESTROL**

On opère comme à l'exemple I, mais en utilisant 1.5 volumes d'un solvant ternaire formé de :
- 94,9 % de méthyléthylcétone
- 5,0 % d'eau
- 0,1 % de Tween 20

On recueille des microcristaux dont la taille s'échelonne de 120 x 40 $\mu$m pour les plus gros à 75 x 50 $\mu$m pour les plus petits.

### EXEMPLE X

**MICROCRISTAUX DE PROMESTRIENE**

On opère comme à l'exemple I, mais en dissolvant une partie de Promestriène dans 4 volumes d'un solvant ternaire composé de :
- 94,9 % d'Ethanol absolu
- 5,0 % d'eau
- 0,1 % de Tween 20

On recueille les cristaux dont la taille s'échelonne de 300 x 100 $\mu$m pour les plus gros à 100 x 50 $\mu$m pour les plus petits.

### EXEMPLE XI

**MICROCRISTAUX DE PROMESTRIENE**

On opère comme à l'exemple précédent, en dissolvant 30 kg de Promestriène dans 3 volumes d'un solvant ternaire composé de :
- 94,8 % de Méthyléthylcétone, soit 85,320 l
- 5,0 % d'eau, soit 4,500 l
- 0,2 % de Tween 20, soit 0,180 l

On refroidit à -10°. Après 15 mn à cette température, on filtre les cristaux formés. On les lave à l'eau et on les sèche en étuve sous pression réduite vers 35°. Les cristaux ainsi recueillis ont une taille qui s'échelonne de 125 x 100 $\mu$m pour les plus gros à 80 x 50 $\mu$m pour les plus petits.

Les produits qui ont servi à cette étude (bruts, broyés et microcristallisés) ont été étudiés par analyse thermique différentielle.

Des études séparées entre un produit brut et broyé (C114 et C114B) entre deux produits broyés (C108B et C114B) et un produit brut et microcristallisé (C109 et C109M) ont donné les résultats suivants :

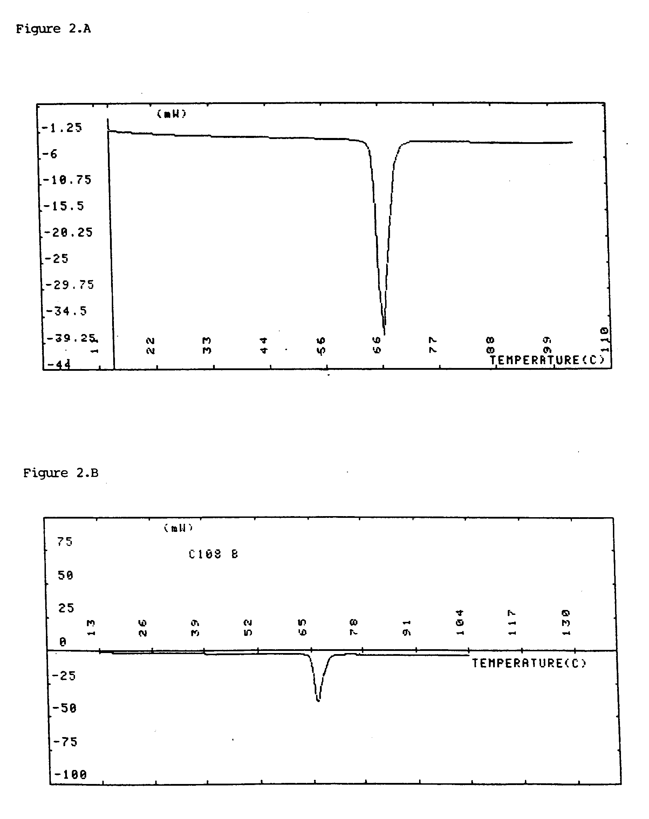

- entre les essais C108B, C114B et C114 dans des conditions expérimentales identiques pour le domaine de température allant de 12° à 142°C, ces trois produits présentent lors de l'analyse calorimétrique différentielle à balayage de température des comportements similaires. (Figures 2,3,4,5 et 6)

Les fusions de ces composés sont semblables entre elles, avec une température se situant aux alentours de 64°C et une enthalpie de fusion située entre 72 et 74 joules/gramme.

Le microcristallin à une température de fusion de 64, 57°C et une enthalpie de fusion située à 73.2 j/g, donc comparables aux autres produits.

## EXEMPLE XII

## MICROCRISTAUX D'ACETATE DE NOMEGESTROL

On a procédé à deux essais de cristallisation, selon le procédé de l'invention, à partir de 30 kg d'acétate de Nomégestrol.

Le produit est dissout dans 4 volumes d'un solvant ternaire formé de :
- 94,9 % de Méthanol
- 5,0 % d'eau
- 0,1 % de Tween 20

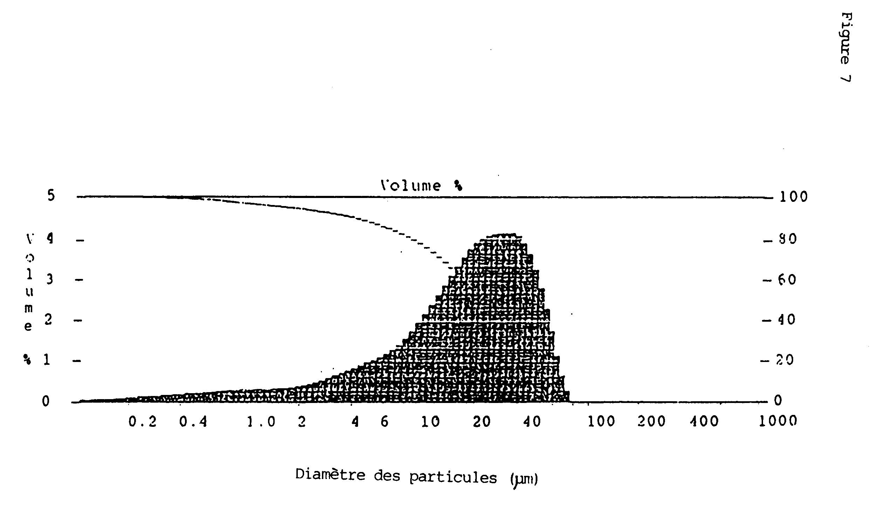

Le produit cristallin recueilli présente la gamme de granulométrie suivante (au granulomètre Laser COULTER LS130) :
- essai 1 : lot 037 MC2 (Figure 7)
- essai 2 : lot 037 MC3

Pour ces deux essais, les résultats sont statistiquement comparables pour une classe granumétrique dont toutes les particules sont < à 100 $\mu$m.

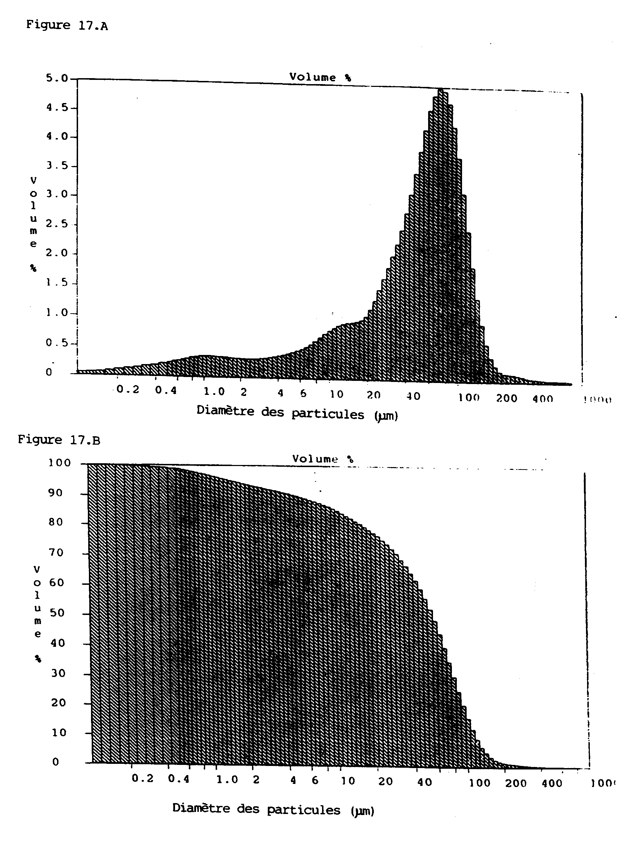

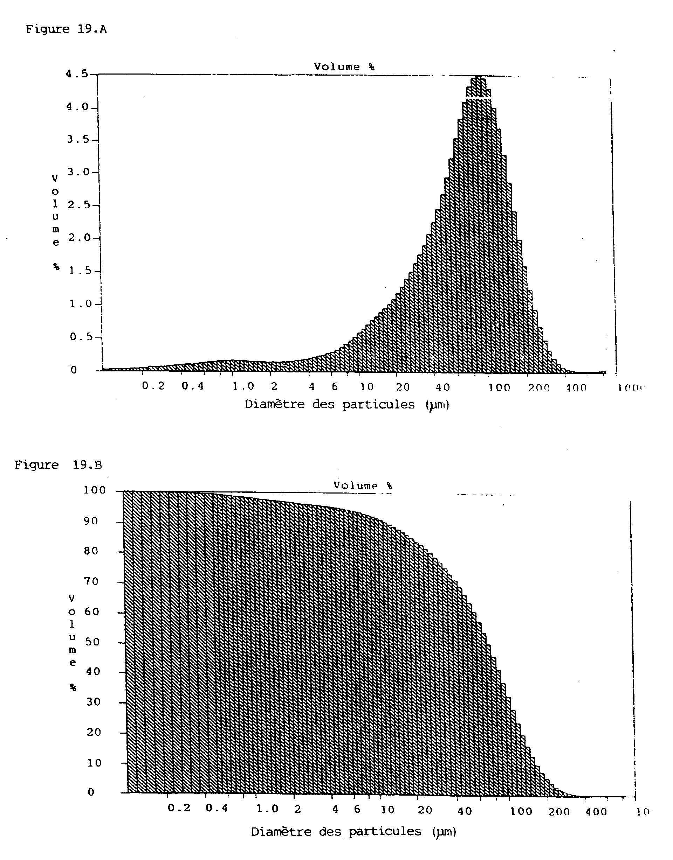

Par broyage de différents lots industriels, on constate une grande irrégularité (voir courbes des lots 23, 27, 33, 35) dans les classes granulométriques. (Figures 17 à 20)

## EXEMPLE XIII

Des essais ont été effectués sur des charges plus importantes de 40, 60 et 90 kg, selon la technique décrite à l'exemple XII et ont donné d'aussi bons résultats.

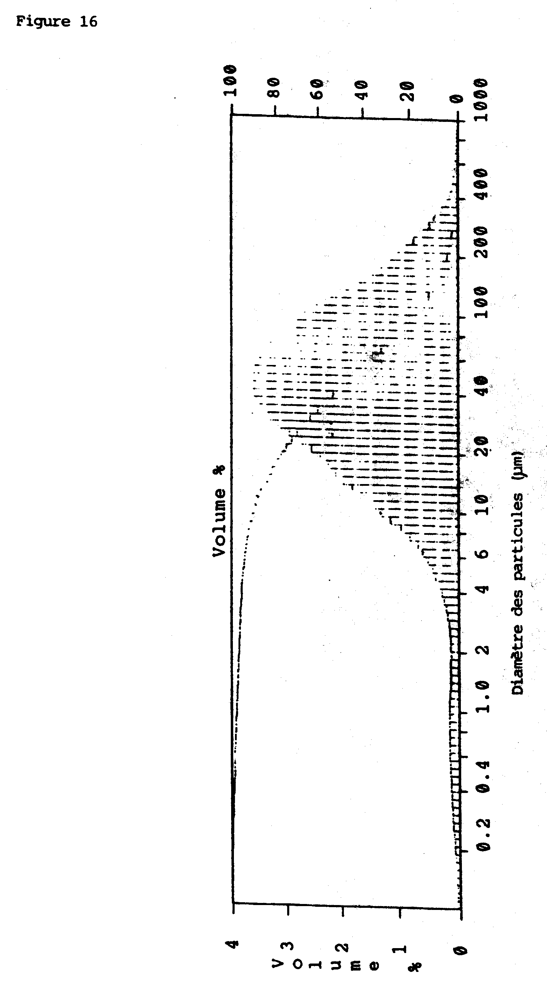

Un essai sur 90 kg (référnce S4-lot 3) a donné l'analyse granulométrique suivante : (voir Figure 16)

## EXEMPLE XIV

## MICROCRISTAUX DE PROGESTERONE

On opère comme dans les exemples précédents en dissolvant le produit au reflux dans 6 volumes d'un solvant formé de :
- 93,4 % d'éthanol
- 6,0 % d'eau
- 0,6 % de Tween 40

On sépare à froid les cristaux formés que l'on lave à l'eau et sèche. Les cristaux ainsi formés vont de 150 x 80 $\mu$m pour les plus gros et 10 x 20 $\mu$m pour les plus petits.

$PF_K$ : 125° ± 2°

$\alpha_D$ (Dioxane) + 170 ± 4°

## EXEMPLE XV

## MICROCRISTAUX DE 17$\beta$-ACETOXY-17$\alpha$-ETHYNYL-5$\alpha$-ANDROSTEN-2

On opère comme dans les exemples précédents en dissolvant le produit au reflux dans 4 volumes d'un mélange composé de :

- 96,0 % d'acétate d'éthyle
- 3,2 % d'eau
- 0,8 % de Tween 20

On isole comme décrit précédemment dans les exemples précédents. Les cristaux ainsi formés ont une granulométrie moyenne de 40 à 60 $\mu$m. PF$_K$ : 125 - 129°

## EXEMPLE XVI

## MICROCRISTAUX D'ANDROSTANOLONE (4-DIHYDROTESTOSTERONE)

On opère comme dans les exemples cités précédemment en dissolvant le produit au reflux dans 7,5 volumes d'un solvant formé de :
- 91,8 % de Méthanol
- 8,0 % d'eau
- 0,2 % de Tween 20

On sépare à froid les cristaux formés que l'on lave à l'eau et sèche. Les cristaux ont une granulométrie moyenne de 120 $\mu$m. PF$_K$ : 182° ± 2° $\alpha_D$ (alcool) : +30 ± 2°

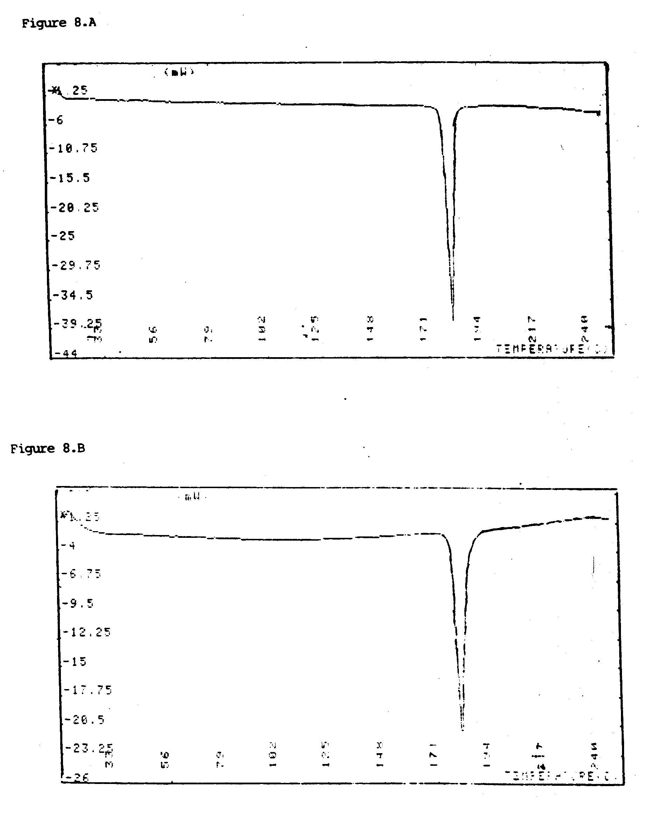

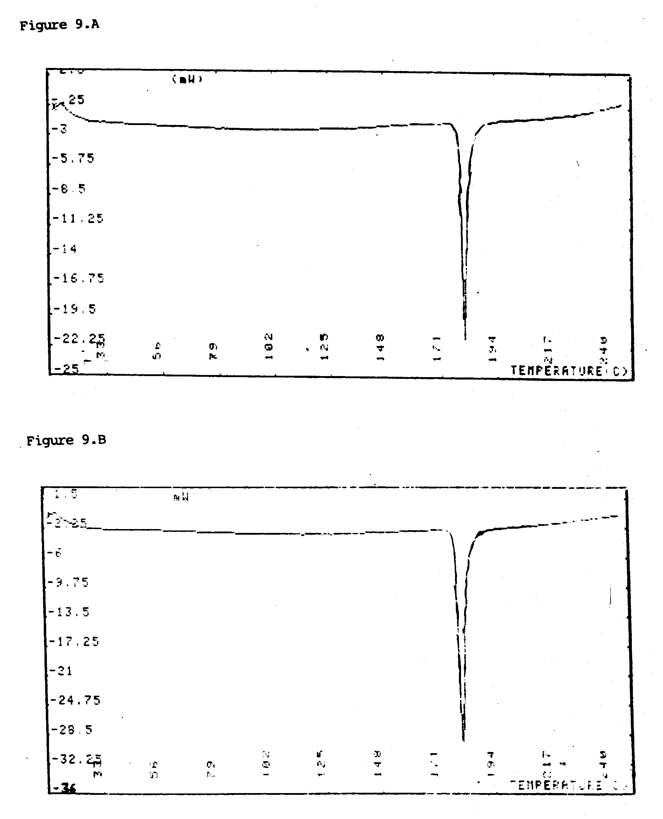

Une étude d'analyse thermique différentielle a été effectuée sur des lots industriels acétate de Nomégestrol avant broyage, après broyage et après microcristallisation :
- échantillons A et B pour l'acétate de Nomégestrol avant broyage (Figure 8)
- échantillons C et D pour l'acétate de Nomégestrol après broyage (Figure 9)
- échantillons E et F pour l'acétate de Nomégestrol après microcristallisation.

L'objet de l'invention : la microcristallisation, est effectuée sur le produit avant broyage.

## RESULTATS :

Les températures d'essai vont de 290 à 525°K. On n'a pas trouvé de transition solide-solide. Les transitions solide-liquide ont été, selon les échantillons, de 178,1 à 179,1°C.

L'enthalpie de transition est d'environ 6,8 joules/gramme.

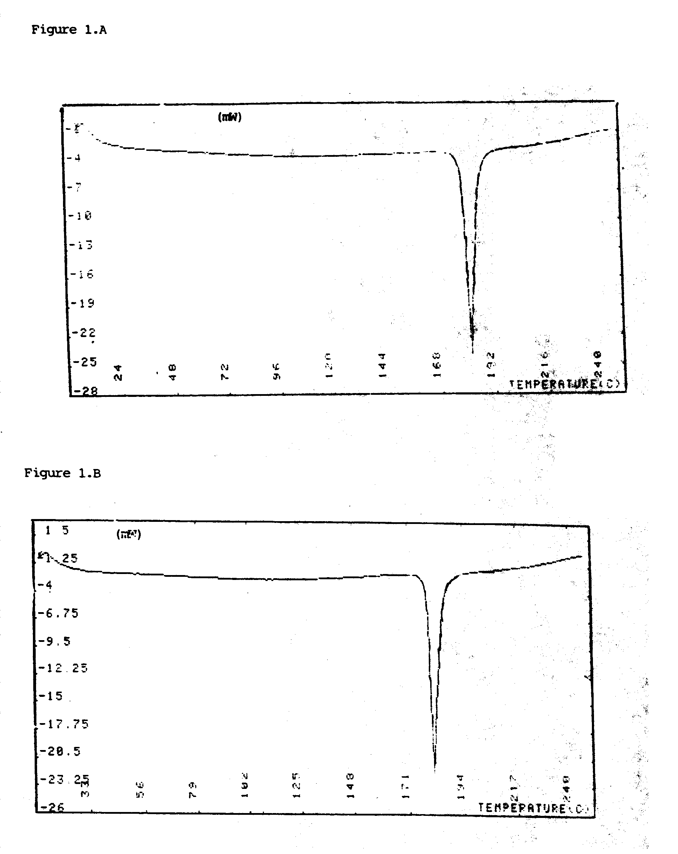

Les écarts de température pour les transitions solide-liquide ont été les plus faibles pour les échantillons E et F provenant du produit microcristallisé selon le procédé revendiqué. (Figure 1)

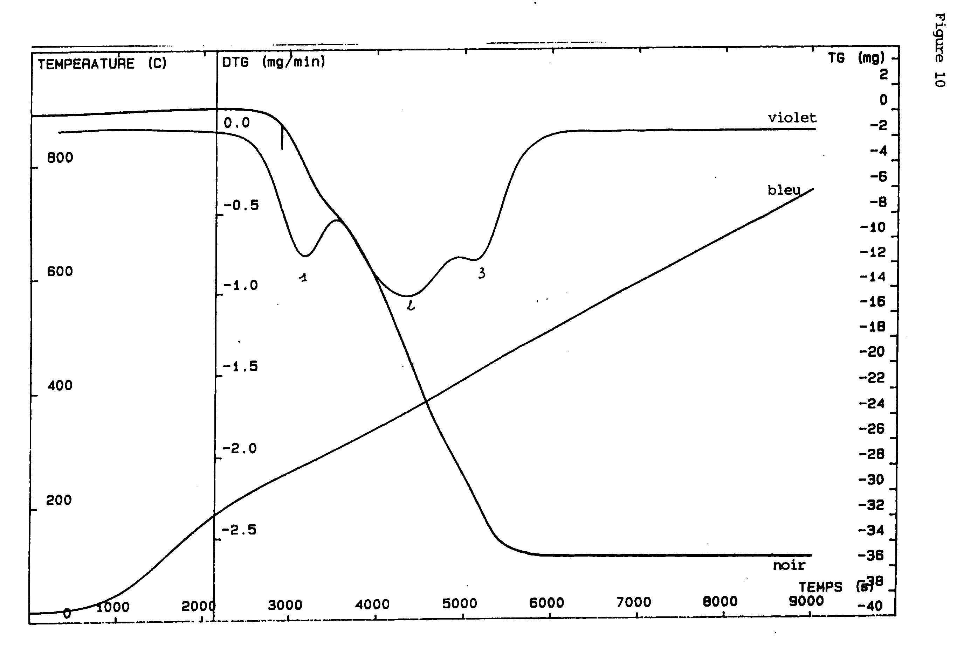

Une analyse thermogravimétrique a été effectuée sur un échantillon (B), produit de départ. Sur ce type de produit aux températures d'essai de 25°C à 700°C, la perte de masse est quasi-totale vers 400°C. (Figure 10)

Il est possible également d'étudier les classes granulométriques obtenues par le procédé de microcristallisation à l'aide d'un analyseur d' image muni d'un logiciel VIDS IV.

Sur le produit recristallisé industriellement selon le procédé de l'exemple XII (référence 037 MC2) on a effectué une étude sur ce type de matériel.

Les résultats sont exprimés en $\mu$m pour les dimensions (paramètre et longueur) et en $\mu$m2 pour les surfaces.

Ce type d'analyse permet de confirmer les résultats obtenus par granulométrie Laser.

Sur un champ de petites particules, on a relevé des particules de 9 à 16 $\mu$mn représentatives de celles observées par granulométrie au Laser.

Une analyse des plus grosses particules a donné des dimensions voisines de 70 $\mu$m, valeur également obtenue au granulomètre Laser.

L'étude chromatographique (HPLC) a permis de démontrer l'influence néfaste du broyage.

Pour l'acétate de Nomégestrol, cette dégradation peut s'évaluer à environ 0,2%.

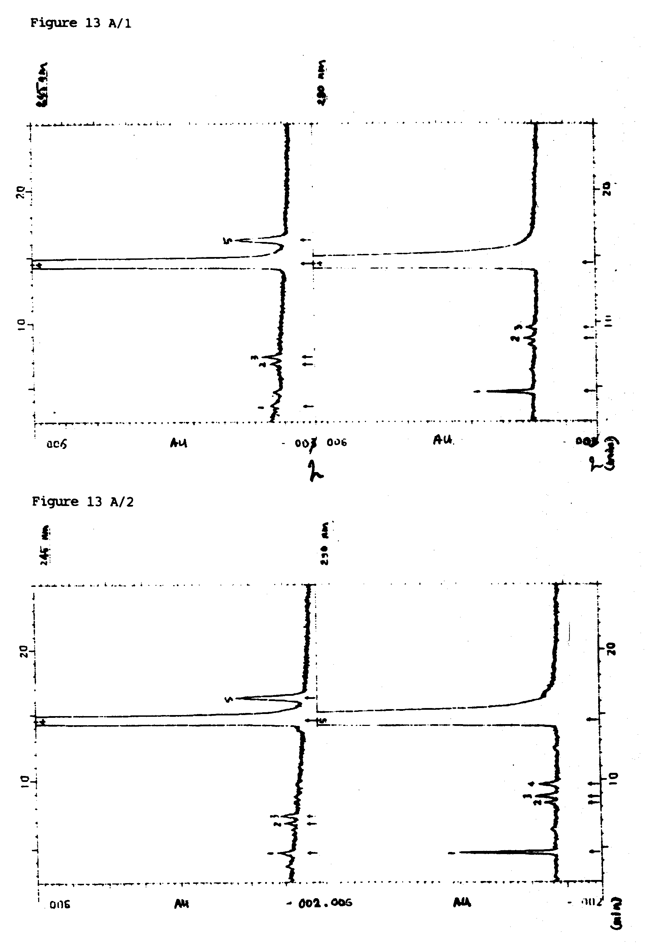

Quelques exemples de lots industriels mettent en évidence ce phénomène :
- Ref. 028 et 031 : lots avant broyage (Figure 13)
- Ref. 028B et 031B : ces mêmes lots après broyage. (Figure 13)

Les analyses par HPLC ont été effectuées à deux longueurs d'ondes différentes (245 et 290 nm) pour mieux séparer les impuretés de type 3-céto delta-4 de celles du 3-céto delta-4,6 pregnadiène.

Pour redédier à cette dégradation, il a été envisagé plusieurs techniques :
- l'une consistait à broyer en refrigérant l'appareillage etl'autre utilisait la technique de cristallisation selon l'invention.

Pour être comparatif et valider la technique selon l'invention, on a mis en pratique ces deux techniques sur un même lot de principe actif.

EP 0 510 167 B1

## SCHEMA DE L'ETUDE

037    Produit sorti de synthèse

037-2    Produit cristallisé normalement

Broyage avec
refrigération

037-2B    037-MC1    037-MC2    037-MC3

Les lots définis 037-MC1 à 3 sont 3 essais de recristallisation sur des unités opératoires de 30 kg pour confirmer la validité de la méthode décrite en classe de granulométrie. (Figures 7,11 et 12)

Les HPLC sur ces produits obtenus ont été effectués dans les mêmes conditions que celles citées précédemment.

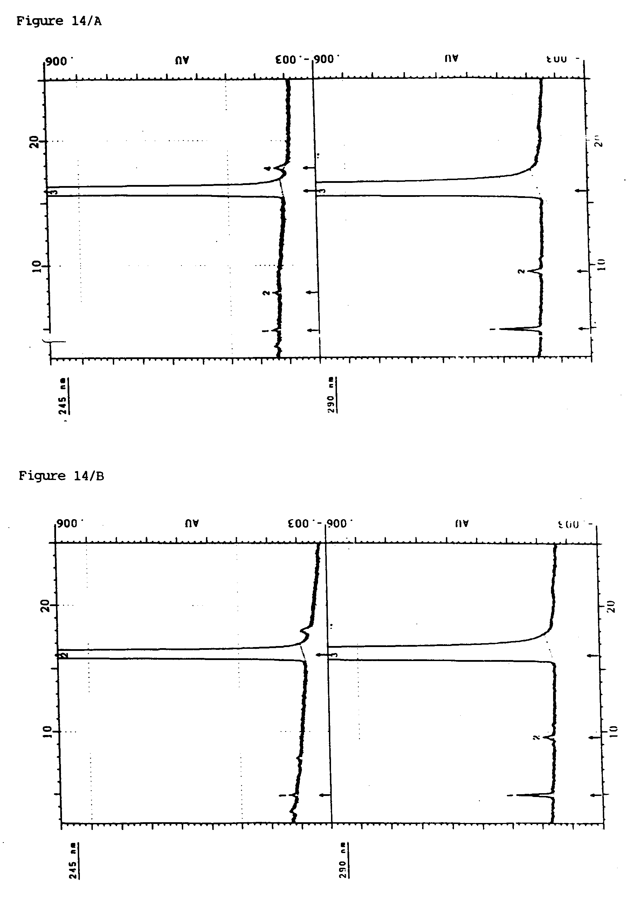

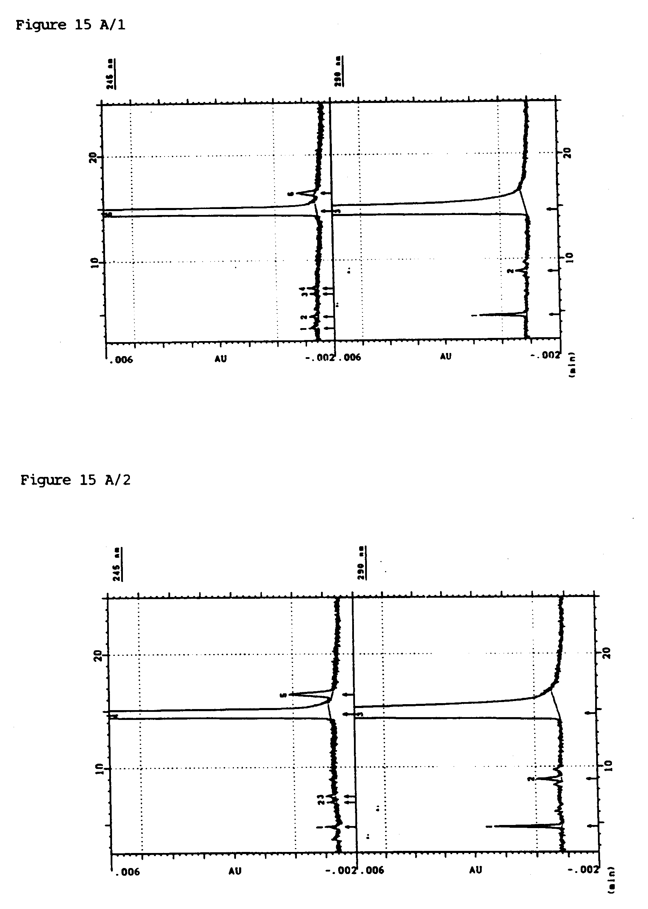

Ces essais démontrent amplement que :
- le broyage même effectué avec refrigération, n'empêche pas le produit de se dégrader (Figure 14)
- la cristallisation, selon le procédé de l'invention, permet d'obtenir un principe actif de meilleure qualité et cela dans une classe granulométrique recherchée sans avoir à recourir à une technique de broyage (Figure 15)

Ce phénomène de dégradation chimique est également constaté sur d'autres produits stéroidiens, à des degrés plus ou moins importants, selon les principes actifs.

Les exemples suivants de formulation illustrent l'utilisation des produits microcristallisés de l'invention et en particulier de composés dérivés du pregnane.

La classe granulométrique mentionnée est la plus représentative du produit (> 80 %).

## EXEMPLE XVII

## COMPRIMES A LIBERATION RETARDEE

| Formule unitaire des dosages différents : | |
|---|---|
| Acétate de Nomégestrol microcristallisé (200 à 300 $\mu$m) | 1,25 à 10,00 mg |
| Aérosil 200 | 0,37 à 0,50 mg |
| Précirol ATO 5 | 1,85 à 2,25 mg |
| Méthocel E.4 | 55,00 à 70,00 mg |
| Avicel PH 101 | 10,00 à 20,00 mg |
| Lactose QSP pour 1 comprimé de | 185,00 à 200,00 mg |

10

### EXEMPLE XVIII

**COMPRIMES A LIBERATION ACCELEREE**

| Formule unitaire des dosages différents : | |
|---|---|
| Acétate de Nomégestrol microcristallisé (< 50 $\mu$m) | 1,25 à 10,00 mg |
| Aérosil 200 | 0,37 à 0,50 mg |
| Précirol ATO 5 | 1,85 à 2,00 mg |
| Avicel PH 102 | 50,00 à 70,00 mg |
| Explotab ou Polyplasdone XL | 5,00 à 25,00 mg |
| Lactose QSP pour 1 comprimé de | 185,00 à 225,00 mg |

### EXEMPLE XIX

**COMPRIMES D'ACETATE DE NOMEGESTROL**

| Formule unitaire des dosages différents : | |
|---|---|
| Acétate de Nomégestrol microcristallisé (200 à 300 $\mu$m) | 1,25 à 10,00 mg |
| Aérosil 200 | 0,37 à 0,50 mg |
| Précirol ATO 5 | 1,85 à 2,25 mg |
| Avicel PH 101 | 55,00 à 70,00 mg |
| Lactose QSP pour 1 comprimé de | 185,00 à 220,00 mg |

Le traitement des résultats par la fonction de distribution de WEIBULL [D.GIBASSIER et Al., STP PHARMA, 1(10), 967-973 (1985)] démontre une différence significative entre ces trois formulations.

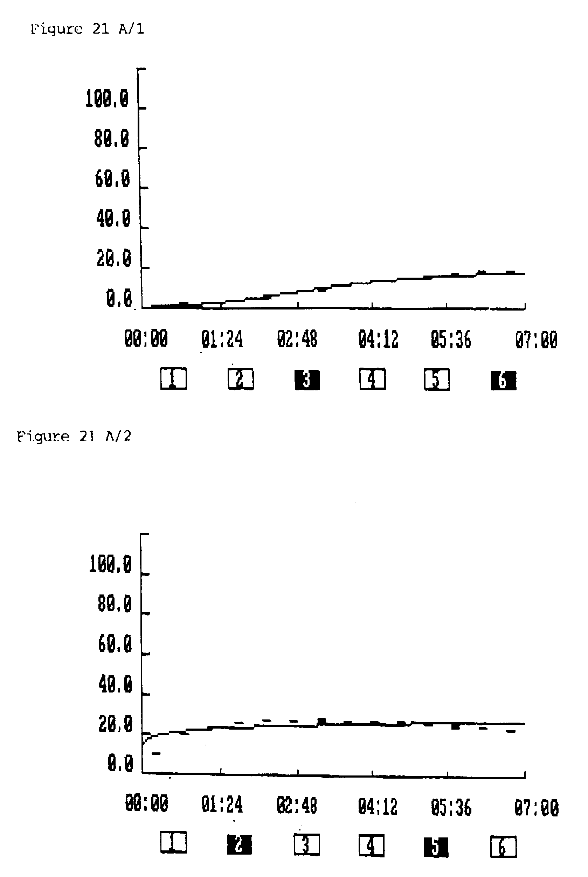

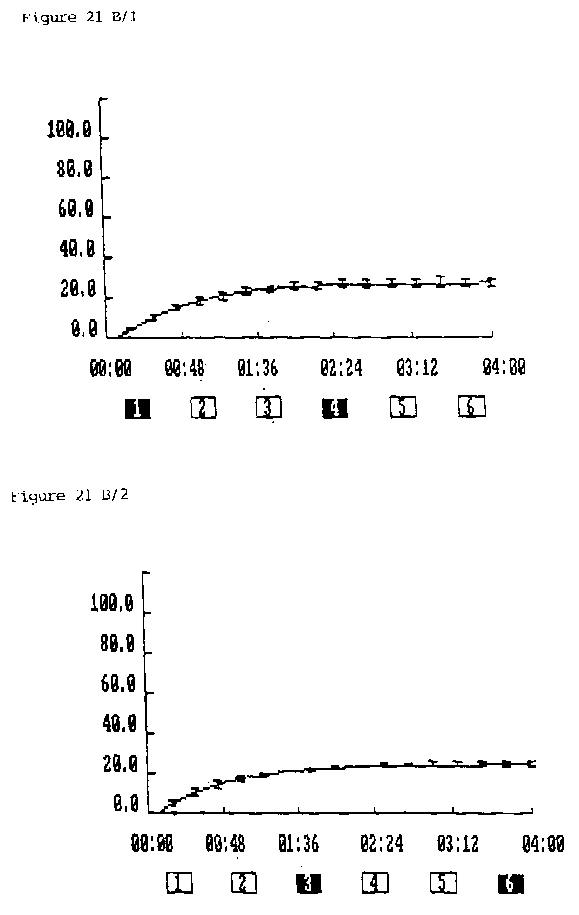

Les formes des courbes de dissolution, représentées par le paramètre $\beta$ de cette fonction, donnent 0.148 pour une forme à libération rapide, 1.015 pour une forme à libération normale et 1.914 pour une forme à libération prolongée; (Figure 21)

Le procédé de l'invention permet donc de réaliser des classes granulométriques d'un principe actif adaptées aux besoins des formes à réaliser.

On constate que ces deux formes pharmaceutiaues, réalisées à partir de lots industriels, ont une lyodisponibilité équivalente.

### EXEMPLE XX

**FORME DEPOT INJECTABLE A BASE DE MEDROXY PROGESTERONE OU DE NOMEGESTROL SOUS FORME D'ACETATES**

| Formule unitaire pour 1 flacon de 5 ml : | |
|---|---|
| Acétate de médroxyprogestérone microcristallisé ou | 500,00 mg |
| Acétate de Nomégestrol microcristallisé (15 à 40 $\mu$m) | |
| Polyéthylène glycol 4000 | 200,00 mg |
| Conservateurs | 0,006 mg |
| Chlorure et citrate de sodium | 0,15 mg |
| Eau pour injectable QSP | 5,00 mg |

**EXEMPLE XXI**

**CAPSULE VAGINALE OU GYNECOLOGIOUE**

| a) Formule unitaire pour 1 capsule : | |
|---|---|
| Progestérone microcristallisée (200 à 300 $\mu$m) | 50 à 500,00 mg |
| Vaseline officinale | 0,200 g |
| Sesquioléate de sorbitol | 0,200 g |
| Perhydrosqualène synthétique | 1,85 g |

Enveloppe sèche :     gélatine, glycérine, stabilisateurs pour 1 capsule molle pesant 2,55 g

| b) Acétate de Nomégestrol microcristallisé | 20,00 mg |
|---|---|
| Witepsol H35 et H37 QSP 1 ovule de | 2,8 g |

**EXEMPLE XXII**

**GEL BIOADHESIF A USAGE CUTANE OU GYNECOLOGIOUE**

| Formule pour 100 g : | |
|---|---|
| Progestérone microcristallisée | 2,0 à 3,0 g |
| Polyéthylène glycol | 4,0 à 6,0 g |
| Polymères carboxypolyvinyliques | 0,5 à 1,0 g |
| Conservateurs | 0,3 mg |
| Triéthanolamine QSP pH 6,5 | |
| Eau purifiée | 100,0 g |

**EXEMPLE XXIII**

**MOUSSE GYNECOLOGIOUE BIOADHESIVE**

| Formule pour 1 flacon doseur (2,5 ml) de 50 g : | |
|---|---|
| Progestérone microcristallisée | 2,0 à 5,0 g |
| Polymère carboxypolyvinylique | 0,5 % |
| Isobutane | 5,5 % |
| Excipient base F25/1 QSP | 50,0 g |

Agiter la suspension avant utilisation
Dose distribuée de 100 à 250 mg.

EP 0 510 167 B1

**EXEMPLE XXIV**

**IMPLANTS**

| Formule pour 100 g de matière à extruder : | |
|---|---|
| Acétate de Nomégestrol | 5,0 g |
| Polyorthocarbonates QSP | 100,0 g |

La température du mélange ne doit pas excéder 185° pour ne pas altérer la forme cristalline du principe actif.

**EXEMPLE XXV**

**DISPOSITIF INTRA-UTERIN AVEC RESERVOIR**

Par réservoir de Silastic de 2.5 à 3.5 cm de long pour une épaisseur de 0.4 à 0.8 mm et de 2 mm de diamètre.

| Pour 100 g de suspension : | |
|---|---|
| Progestérone microcristallisée (80/250 $\mu$m) | 0,600 à 1,0 g |
| en suspension dans : | |
| Agent de suspension | 0,5 g |
| Perhydrosqualène synthétique QSP | 100,0 g |

**EXEMPLE XXVI**

**PATCH**

| Contenu du réservoir - Préparation pour 100 g : | |
|---|---|
| Acétate de nomégestrol microcristallisé (80/250 $\mu$m) | 0,5 g |
| Polymère carboxypolyvinylique | 0,2 g |
| Silice colloïdale | 0,2 g |
| Huile de silicone QSP | 100,0 g |

A partir de substances dérivées des estranes :

**EXEMPLE XXVII**

**COMPRIMES**

| Formule unitaire : | |
|---|---|
| Estradiol microcristallisé (10/50 $\mu$m) | 1,0 à 2,0 mg |
| Kollidon 25 | 10,0 à 20,0 mg |
| Kollidon 90 | 5,0 à 10,0 mg |
| Avicel Ph 102 | 25,0 à 50,0 mg |
| PEG 6000 | 1,0 à 2,0 mg |
| Aérosil 200 | 1,0 à 2,0 mg |
| Précirol ATO 5 | 1,5 à 3,0 mg |
| Polyplasdone XL | 2,5 à 5,0 mg |
| Lactose QSP pour 1 comprimé | 185,0 à 220,0 mg |

**EXEMPLE XXVIII**

**GELS BIOADHESIFS D'ESTRADIOL OU DE PROMESTRIENE**

| Formule pour 100 g de gel : | |
|---|---|
| Estradiol ou Promestriène microcristallisé | 1,0 à 2,0 g |
| Propylène glycol | 5,0 à 10,0 g |
| Polymères carboxypolyvinyliques | 0,5 à 1,0 g |
| Conservateurs | 0,3 mg |
| Triéthanolamine QSP pH 6-6,5 | |
| Eau purifiée QSP | 100,0 g |

**EXEMPLE XXIX**

**CAPSULE VAGINALE**

| Formule pour 1 capsule : | |
|---|---|
| Estradiol microcristallisé | 1,0 mg |
| Labrafil M 1944 CS | 0,5 g |
| Perhydrosqualène | 1,3 g |

Enveloppe sèche :     gélatine, glycérine, stabilisateurs pour 1 capsule molle de 2,1 g

**EXEMPLE XXX**

**PATCH**

| Contenu du réservoir - Préparation pour 100 g : | |
|---|---|
| Estradiol microcristallisé (80/100 μm) | 0,5 à 1,0 g |
| Aérosil | 0,5 g |
| Perhydrosqualène synthétique QSP pour | 100,0 g |

A partir de substances dérivées des androstanes :

**EXEMPLE XXXI**

**FORME COMPRIMES**

| Formule unitaire pour 1 comprimé de 380 mg : | |
|---|---|
| 17$\beta$-acétoxy 17$\alpha$-éthynyl 5$\alpha$-androsten-2-microcristallisé | 20,0 mg |
| Avicel PH 101 | 91,20 mg |
| Aérosil | 0,45 mg |
| Précirol ATO 5 | 7,60 mg |
| Explotab | 4,30 mg |
| Lactose | 256,45 mg |

Cette forme a une meilleure biodisponibilité que celle décrite précédemment dans la littérature.

**EXEMPLE XXXII**

**GEL GYNECOLOGIOUE**

| Formulation pour 100 g : | |
|---|---|
| Androstanolone (DHT) microcristallisé | 2,50 g |
| Propylène glycol | 2,50 g |
| Transcutol | 5,00 g |
| Conservateurs | 0,08 g |
| Agent viscosifiant (TEA) | 0,25 g |
| Polymères carboxypolyvinyliques | 1,50 g |
| Eau purifiée QSP | 100,00 g |

**EXEMPLE XXXIII**

**CAPSULE ORALE**

| Heptylate de Testostérone microcristallisé | 50,00 mg |
|---|---|
| Acide oléique QSP pour 1 capsule | 250,00 mg |

Envoloppe : gélatine, conservateurs, glycérine
A partir de substances dérivées des 21-hydroxy prégnènes :

15

### EXEMPLE XXXIV

**COMPRIMES ORAUX**

| Formule unitaire pour 1 comprimé : | |
|---|---|
| Prédnisone microcristallisée (80/150 $\mu$m) | 2,50 mg |
| Avicel PH 102 | 50,00 mg |
| Aérosil | 1,80 mg |
| Précirol ATO 5 | 2,00 mg |
| Lactose QSP 1 comprimé | 128,70 mg |

### EXEMPLE XXXV

**COMPRIMES ORAUX**

| Formule unitaire pour 1 comprimé : | |
|---|---|
| Prédnisone microcristallisée (80/150 $\mu$m) | 0,50 mg |
| Avicel PH 102 | 50,00 mg |
| Aérosil | 1,70 mg |
| Précirol ATO 5 | 2,00 mg |
| Lactose QSP 1 comprimé | 130,00 mg |

### EXEMPLE XXXVI

**POUR APPLICATION CUTANEE**

| Formulation pour 100 g : | |
|---|---|
| Acétate de Dexaméthasone microcristallisé (100 à 325 $\mu$m) | 0,05 à 0,10 g |
| Polyéthylène glycol | 5,00 g |
| Polymères carboxypolyvinyliques | 1,00 g |
| Triéthanolamine QSP pH 6,5 | |
| Eau purifiée QSP | 100,00 g |

EP 0 510 167 B1

**EXEMPLE XXXVII**

**SUSPENSION INJECTABLE**

| Formulation unitaire pour 1 ampoule de 2 ml : | |
|---|---|
| Acétate de Dexaméthasone microcristallisé ($< 80\ \mu$m) | 10,00 mg |
| Soluté de suspension : | |
| Polysorbate 80<br>Carboxyméthylcellulose sodique<br>Chlorure de sodium<br>Eau p.p.i QSP | 0,015 g<br>0,010 g<br>0,010 g<br>2,00 ml |

**LEGENDES DES FIGURES**

**La figure 1A/21** décrit la détermination de l'état de transition vitreux

- début du pic = 171,4°
  $K_1$ = 2.474
  $S(K_1)$ = -3,1993
- fin du pic = 201,1°C
  $K_2$ = 2.920
  $S(K_2)$ = -2,7929
Enthalpie = -1,182E + 003m J
  ou    -1,598E + 001 cal/g
  ou    -6,679E + 001 J/g
Pic endothermique
  TP = 181,519107667
Sommet de la température du pic : 179,7°C

Détermination de l'état de transition vitreux

  Gamme de température pour la détermination Tg
  - température de début = 171,5°C
  - température de fin = 181,9°C
les trois températures vitreuses sont : 173,8° - 178,3° et 179,4°C Pente = -5,74403141606

**La figure 1B/21** décrit la détermination de l'état de transition vitreux

- Début du pic = 172,6°C
  $K_1$ = 2.401
  $S(K_1)$ = -2,1753
- Fin du pic = 198,5°C
  $K_2$ = 2.791
  $S(K_2)$ = -1,8858
Enthalpie : -9,665E + 002 mJ
  ou    -1,639E + 001 cal/g
  ou    -6,855E + 001 J/g
Pic endothermique TP = 181,3857774333
Sommet de la température du pic = 179,6°C

Détermination de l'état de transition vitreux

  Gamme de températures pour la détermination de Tg
  - température de début = 174,2°C

17

- température de fin = 181,1°C

Les trois températures vitreuses sont : 176,7° - 178,8° et 179,7°C Pente = 7,91818986688

**La figure 2A/21** donne :

- la température initiale : 285,2°K
- la température finale : 379,3°K
- la vitesse de balayage : 2,00 C/mn
- la gamme d'amplification : 1.000 mV
- la masse de l'échantillon : 37,200 mg
- la vitesse de saisie de l'échantillon : 2,08s
- la conservation : 1.357 points

**La figure 2B/21** donne la détermination de l'état de transition vitreux

Gamme de températures pour la détermination de Tg :
- température de début : 61,79°C
- température de fin : 66,63°C

Les trois températures vitreuses ont été : 64,0° - 64,79° et 65,38°C Variation de Cp = 6,64 cal/g en C

Intégration avec une ligne de base linéaire

- début du pic = 62,06°C
- fin du pic = 74,23°C

Démarrage de la température = 64,89°C

Enthalpie = -0,27672E + 004 m J

    ou    -0,74389E + 002 J/g

    ou    -17796E + 002 cal/g

    ou    -0,66202 + 0,03 mcal

Pic endothermique

Sommet de la température du pic = 66,56°C

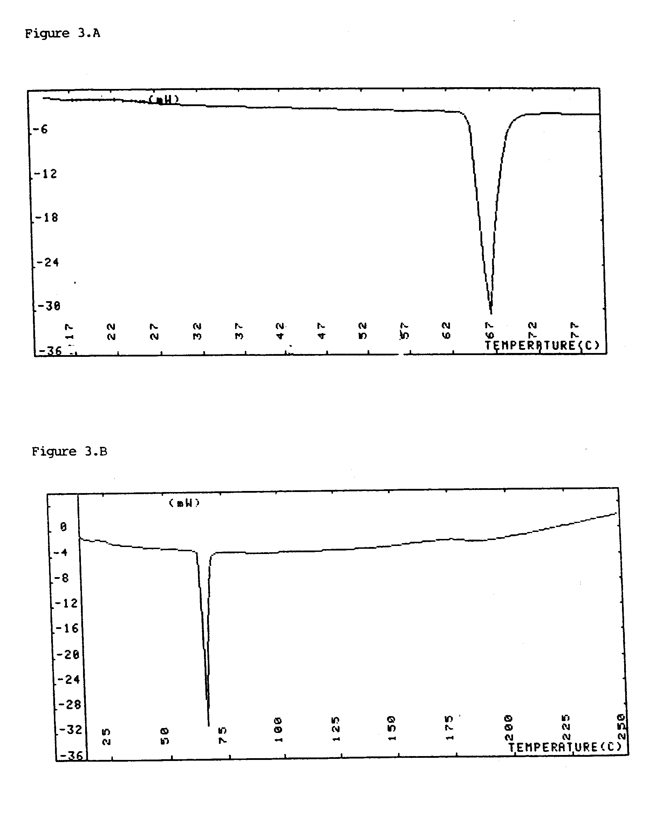

**La figure 3A/21** (ref.DEF C114B) donne :

- la température initiale : 285,2°K
- la température finale : 525,0°K
- la vitesse de balayage : 2,00 C/mn
- la gamme d'amplification : 1.000 mV
- la masse de l'échantillon : 25,400 mg
- la vitesse de saisie de l'échantillon : 3,68s
- la conservation : 1.955 points

**La figure 3B/21** donne la détermination de l'état de transition vitreux

Gamme de températures pour la détermination de Tg
- température de début = 62,29°C
- température de fin = 66,57°C

Les trois températures vitreuses ont été : 64,1° - 64,55° et 64,98°C

Variation de Cp = 4,56 cal/g en C

Intégration avec une ligne de base linéaire

- début du pic = 62,16°C
- fin du pic = 75,38°C

Température de démarrage = 64,62°C

Enthalpie = -0,18837E + 004 mJ

ou      -0,45064E + 003 mcal
ou      -0,74161E + 002 J/g
ou      -0,17742E + 002 cal/g

Pic endothermique

Sommet de la température du pic = 66,44°C

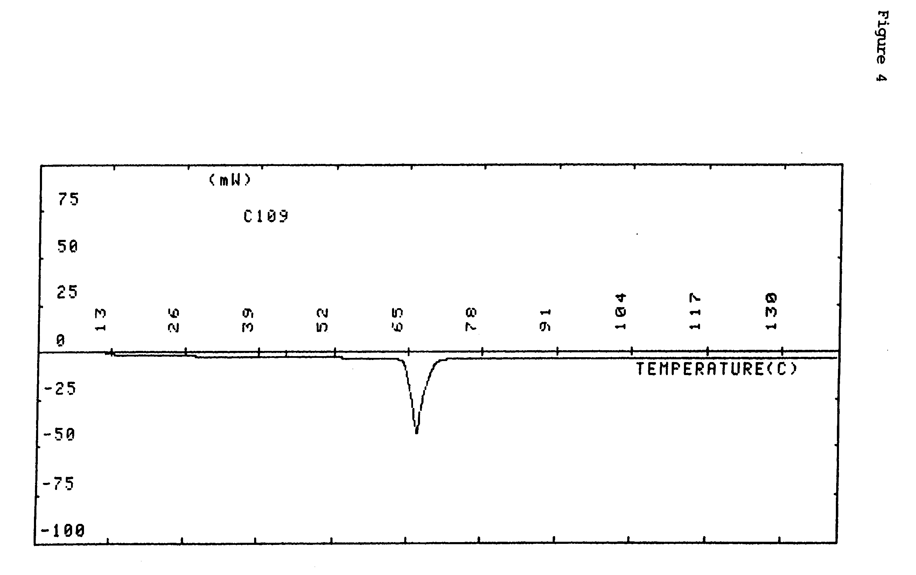

**La figure 4/21** donne :

- la température initiale : 285,2°K
- la température finale : 415,2°K
- la vitesse de balayage : 2,00 C/mn
- la gamme d'amplification : 1.000 mV
- la masse de l'échantillon : 42,200 mg
- la vitesse de saisie de l'échantillon : 2,08s
- la conservation initiale : 1.875 points
- la conservation finale : 1.875 points dans le dossier

Intégration avec une ligne de base linéaire

- début du pic = 61,16°C
- fin du pic = 73,61°C
Démarrage de la température = 64,75°C
Enthalpie = -0,30705E + 004 mJ
     ou      -0,73458E + 003 mcal
     ou      -0,72762E + 002 J/g
     ou      -0,17407E + 002 cal/g

Pic endothermique

Sommet de la température du pic = 66,69°C

Détermination de l'état de transition vitreux

Gamme de température pour la détermination de Tg
- température de début = 61,16°C
- température de fin = 66,63°C
Les trois températures vitreuses ont été : 63,7° 64,22° et 65,24°C Variation de Cp = 5,17 cal/g.C

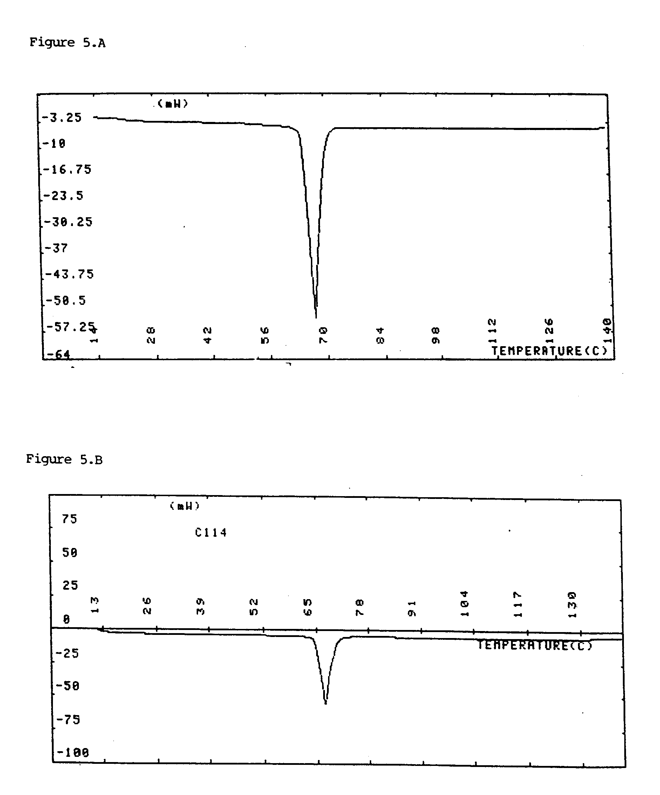

**La figure 5A/21** (Ref.DEE C.114) donne :

- la température initiale : 285,2°K
- la température finale : 415,2°K
- la vitesse de balayage : 2,00 C/mn
- la gamme d'amplification : 1.000 mV
- la masse de l'échantillon : 61,600 Mg
- la vitesse de saisie de l'échantillon : 2,08s
- la conservation : 1.875 points

**La figure 5B/21** donne la détermination de l'état de transition vitreux

Gamme de températures pour la détermination de Tg
- température de début = 57,84°C
- température de fin = 67,04°C
Les trois températures vitreuses ont été : 63,6° 64,31° et 65,52°C
Variation de Cp = 5,24 cal/g en C

Intégration avec une ligne de base linéaire

- début du pic = 57,08 °C
- fin du pic = 77,00 °C

Température de démarrage = 64,78 °C
Enthalpie = -0,45478E + 004 mJ
  ou  -0,10879E + 004 mcal
  ou  -0,73828E + 002 J/g
  ou  -0,17662E + 002 cal/g

Pic endothermique

  Sommet de la température du pic = 66,90 °C

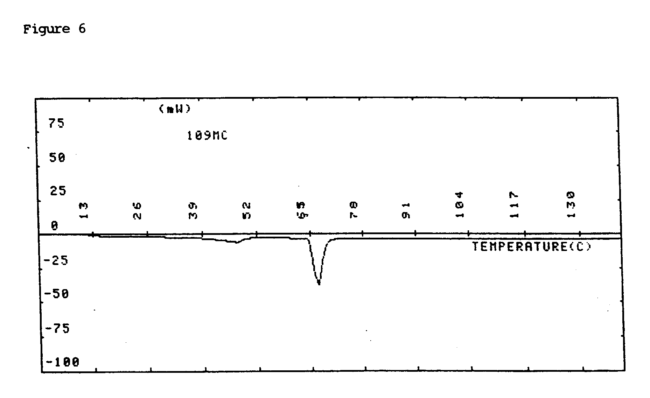

**La figure 6/21** donne :

- la température initiale : 285,2 °K
- la température finale : 415,2 °K
- la vitesse de balayage : 2,00 C/mn
- la gamme d'amplification : 1.000 mV
- la masse de l'échantillon : 35,800 mg
- la vitesse de saisie de l'échantillon : 2,08s
- la conservation : 1.875 points

Détermination de l'état de transition vitreux

  Gamme de températures pour la détermination de Tg :
- température de début = 61,65 °C
- température de fin = 67,04 °C

Les trois températures vitreuses ont été : 64,4 °, 64,57 ° et 65,59 °C
Variation de Cp = 7,48 cal/g en C

Intégration avec une ligne de base linéaire

- début du pic = 61,65 °C
- fin du pic = 73,26 °C

Température de Démarrage : 64,65 °C
Enthalpie = -0,26606E + 004 mJ
  ou  -0,62695E + 003 mcal
  ou  -0,73203E + 002 J/g
  ou  -0,17512E + 002 cal/g

Pic endothermique

  Sommet de la température du pic = 66,90 °C

**La figure 7/21** fournit :
(28.08.1990 - 9h10)

  Analyse de la taille de particules par Coulter[R] LS Ref. A00890.S 18
Nom du dossier : A0 890 S18 Groupe ID : A0 890
Echantillon ID : TX 066 Lot 037 MC 2
Numéro d'essai : 6
Opérateur : A.P.C SIMM
Commentaires - dispersion : glycérol et ultra-sons dans Coultronic (Fr)
Temps du début : 10 h 42 - le 25 Août 1990
Longueur de l'essai : 90 s
Obscurcissement : 6%

Obscurité PIDS : 30%
Modèle optique : Fraunhofer PIDS compris
PC : version 1:10 à 13h07 - Vendredi 2 Mars 1990
Statistiques de volume (arithmétique) A0 890.S18
Calculs de 0,10 $\mu$m à 834,40 $\mu$m
Volume : 100,0 %
Moyenne 22,49 $\mu$m limites de confiance à 95% 19,42-25,56 $\mu$m
Médiane 19,72 $\mu$m déviation standard : 15,67 $\mu$m
Rapport moyenne/médiane : 1,141
Variance 245,5 $\mu$m$^2$
Mode : 29,60
Coefficient de variation : 69,66 %
Obliquité : 7,016e - 0,01 oblique vers la droite
Kurtosis : -1,088e - 0,01 Platykurtic

| % > | 10,00 | 25,00 | 50,00 | 75,00 | 90,00 |
|---|---|---|---|---|---|
| Taille en $\mu$m : | 45,20 | 32,64 | 19,72 | 10,10 | 3,771 |

**La figure 8A/21** :

Intégration avec une ligne de base linéaire

1. Démarrage du pic = 173,9 ° C
$K_1$ = 2.490
Pic endothermique TP = 181,881617
Sommet de la température du pic = 180,1 ° C

Echantillon A

**La figure 8B/21**

2. Sommet de la température du pic = 180,2 ° C
Détermination de l'état de transition vitreux
Gamme de températures pour la détermination de Tg :
- température initiale = 169,5 ° C
- température finale = 181,5 ° C
Les trois températures vitreuses ont été : 176,4, 178,1 et 179,5 ° C Pente = -6,4818199304

**La figure 9A/21**

Echantillon C

Gamme de températures pour la détermination de Tg :
- température initiale : 173,5 ° C
- température finale : 180,5 ° C
Les trois températures vitreuses ont été : 177,2, 178,9 et 179,4 ° C
Pente = -11,8392024107
TP = 181,252441
Sommet de la température du pic = 179,0 ° C

**La figure 9B/21**

Echantillon D

Détermination de l'état de transition vitreux

Gamme de températures pour la détermination de Tg :
- température initiale = 173,5 °C
- température finale = 181,0 °C

Les trois températures vitreuses ont été : 178,0 - 179,1 et 179,7 °C Pente = -21,4280794192

**La figure 10/21**

Cette perte de masse s'effectue en 3 étapes (DTG mg/mn) #54/55 mn 72/74 mn et 87/88 mn).
La perte totale constatée est de 36 mg, soit 94,8% de l'échantillon.
Courbe noire     : perte de masse
bleue                 : température de l'essai
violette              : DTG (mg/mn)

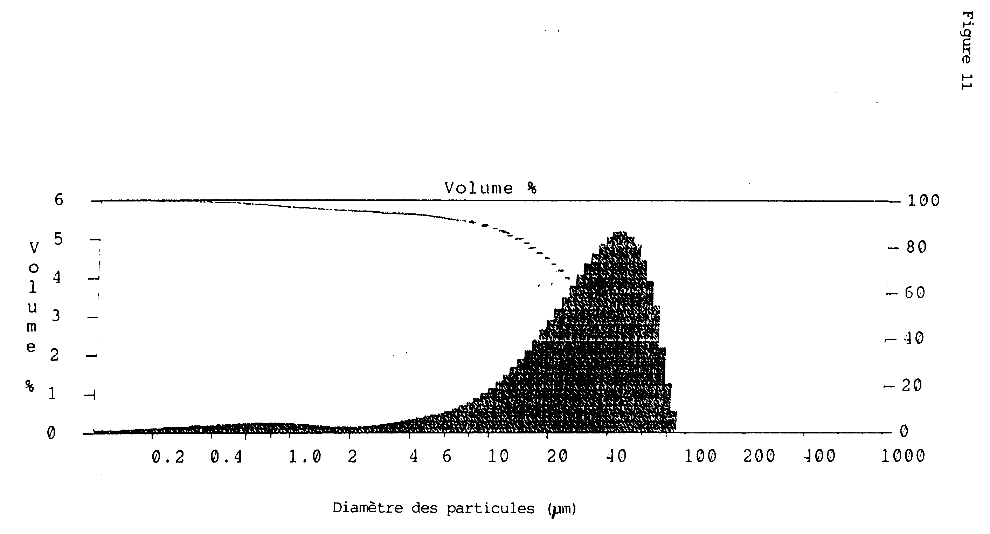

**La figure 11/21**

Analyse de la taille des particules par Coulter[P] LS 9h39 - 28 Août
1990 - A0 889.S.01
Nom du dossier : A0 889.S01
Groupe ID : A0 889
Echantillon ID : TX 066 lot 037 MC1
Nombre d'essai : 1
Opérateur APC Simm
Commentaires : Dispersion Nonidet et Ultra-sons
Coulter France - Nomegestrol Acetate
Heure de démarrage : 11h55 - 24 Août 1990
Durée de l'essai : 91 sec.
Obscurcissement : 5%
Obscurité PIDS : 23%
Modèle optique : Fraunhofer PIDS compris
PC : version 1.10 13h07 Vendredi 2 Mars 1990
Statistiques de volume (arithmétique) A0 889.S
Calculs de 0,10 $\mu$m à 834, 40 $\mu$m
Volume : 100,0 %
Moyenne : 32,93 $\mu$m
Médiane : 31,12 $\mu$m
Rapport moyenne/médiane : 1,058
Mode : 44,70 $\mu$m
Limites de confiance à 95% : 29,02-36,84 $\mu$m
Deviation standard : 16,96 $\mu$m
Variance : 398,4 $\mu$m$^2$
Coefficient de variation : 60,61 %
Obliquité : 3,640e - 001 oblique vers le droite
Curtosis : 5,857e - 001 Platykurtique

| % | 10,00 | 25,00 | 50,00 | 75,00 | 90,00 |
|---|---|---|---|---|---|
| Taille en $\mu$m | 61,21 | 47,02 | 31,12 | 17,27 | 7,31 |

EP 0 510 167 B1

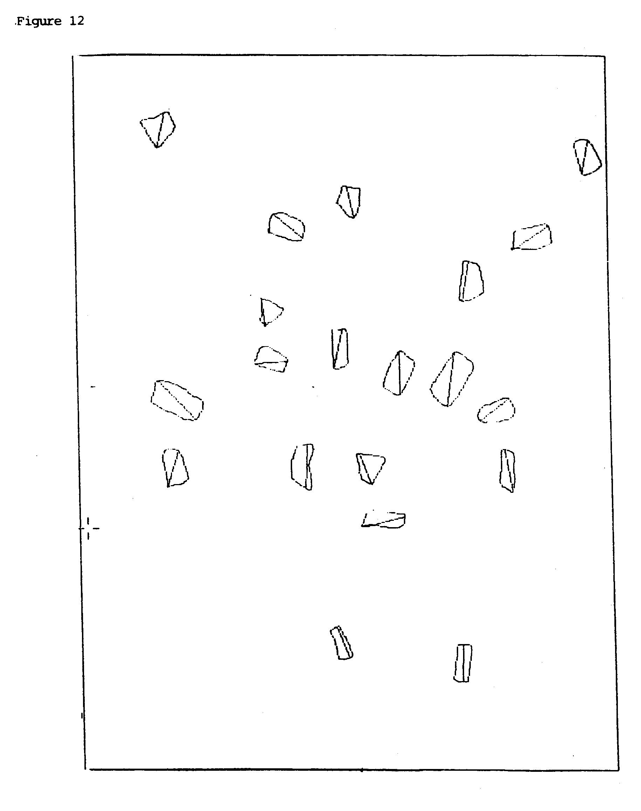

**La figure 12/21** montre l'étude au microscope d'un échantillon d'acétate de Nomegestrol (TX 066)

Echantillon MC 2
Surface du champ : 30.543,81
Calibrage : 0,2717 mcm/pixel
Champ : 1 Classe : 2

| Caractéristique | Surface | Périmètre | Dimension en longueur |
|---|---|---|---|
| 1 | 60.181 | 34.189 | 13.055 |
| 2 | 96.954 | 38.947 | 14.827 |
| 3 | 45.634 | 26.169 | 9.2551 |
| 4 | 69.116 | 33.109 | 12.772 |
| 5 | 58.409 | 30.959 | 12.107 |
| 6 | 53.277 | 29.411 | 10.818 |
| 7 | 37.807 | 28.331 | 12.375 |
| 8 | 49.732 | 29.488 | 11.273 |
| 9 | 59.295 | 29.303 | 10.788 |
| 10 | 48.292 | 27.650 | 9.6381 |
| 11 | 49.474 | 27.094 | 10.229 |
| 12 | 39.727 | 27.849 | 11.145 |
| 13 | 57.818 | 31.689 | 11.848 |
| 14 | 32.490 | 24.579 | 9.6381 |
| 15 | 44.231 | 25.782 | 9.4914 |
| 16 | 29.832 | 22.428 | 7.9550 |
| 17 | 102.086 | 40.780 | 15.910 |
| 18 | 46.188 | 30.739 | 13.058 |
| 19 | 47.443 | 30.379 | 12.261 |
| 20 | 50.877 | 28.224 | 10.233 |
| Total | 1078.86 | 597.098 | 228.675 |
| Moyenne | 53.943 | 29.855 | 11.434 |
| Déviation standard | 18.281 | 4.4267 | 1.9422 |

**Les figures 13A.1/21 - 13A.2/21 - 13B.1/21 et 13B.2/21**

NMA 028-028B-031 et 031B Eau 991
028001.DT3 : 30 Mai 1991 13h50
Echelle des Y : 0,008 AU/FS
Temps d'échantillonage : 21 msec. 4
Sens : normal
Résolution 3 $\mu$m
Gamme de temps : 2.4 à 25 mn
Intervalle : 1 sec
Ligne de base : en dehors
Adoucissement : 5 points
Dérivation : 0,002 AU/mn
Largeur : 0,001 mn
Double durée : 30 mn
Nom de l'échantillon : TX (1-00-1)
Vitesse du papier : 4 mm/mn
Colonne ultra-sphère ODS : 4,6 mm ID x 250 mm
Matériau de remplissage : C.18
Phase mobile : acétonitrile 360 MeOH 240 H 20 400
Vitesse d'écoulement : 1,3 ml/mn
Pression 2300 PSI
Pente 0,001 AU/mn
Hauteur : 0,0002 AU

23

Surface minimale : 0,00002 AU/mn
PIC négatif : en dehors

**La figure 16/21**

Analyse de la taille des particules au Coulter[(R)] LS
Nom du dossier : S4-3 Ø2 Groupe ID : 881 # 91
Echantillon ID : TX 066 Ref. S4 Lot 3
Numéro d'essai : 2
Opérateur : R.J.B.C
Commentaires - milieu : eau dispersion : 1% v/v NONIDET dans $DH_2O$ et U/S ACETATE DE NOMEGESTROL Ech. Théramex via France
Temps du début : 15 h 37 - le 24 Juin 1991
Longueur de l'essai : 91 s
Obscurcissement : 8%
Obscurité SDIP : 54%
Modèle optique : Fraunhofer PIDS compris
PC : version 1.44 à 14h20 - Mercredi 30 Janvier 1991
Statistiques de volume (géométrique) S4-3.S02
Calculs de 0,10 $\mu$m à 900,00 $\mu$m
Volume : 100,0 %
Moyenne 34,76 $\mu$m limites de confiance à 95% 27,20-44,41 $\mu$m
Médiane 40,33 $\mu$m déviation standard : 1.251
Rapport moyenne/médiane : 0,862
Variance 1.564
Mode : 42,62 $\mu$m
Coefficient de variation : 35,25 %
Obliquité : -1.270 oblique vers la gauche
Kurtosis : 3,027 Leptokurtique

| % > | 10,00 | 25,00 | 50,00 | 75,00 | 90,00 |
|---|---|---|---|---|---|
| Taille en $\mu$m : | 134.6 | 77.82 | 40.33 | 19.43 | 8.917 |

**La figure 17/21**

Analyse de la taille des particules par Coulter[R] LS 16h24 11 Mai 1990
023 S 03
Nom du dossier : 023 S 03
Groupe ID : 023
Echantillon ID : TX 066 023
Nombre d'essais : 3
Opérateur : Martine JULIN
Commentaires : dispersant Coulter avec anti-mousse
Heure de démarrage : 13h33 9 Mai 1990
Durée de l'essai : 61 sec.
Obscurcissement : 9%
Modèle optique : Fraunhofer
PC Version : 1.10 13h07 Vendredi 2 Mars 1990
Statistiques de volume (arithmétique) 023.S 03
Calculs de 0,10 $\mu$m à 834,40 $\mu$m
Volume : 100,0%
Moyenne : 59,70 $\mu$m
Médiane : 53,75 $\mu$m
Rapport moyenne/médiane : 1,111
Mode : 73,31 $\mu$m
Limite de confiance à 95% : 49,10 - 70,30 $\mu$m

Déviation standard : 54,07 $\mu$m

Variance : 2923 $\mu$m$^2$

Coefficient de variation : 90,57%

Obliquité : 3,930c - 000 oblique vers la droite

Kurtosis : 3,530c - 001 Platykurtique

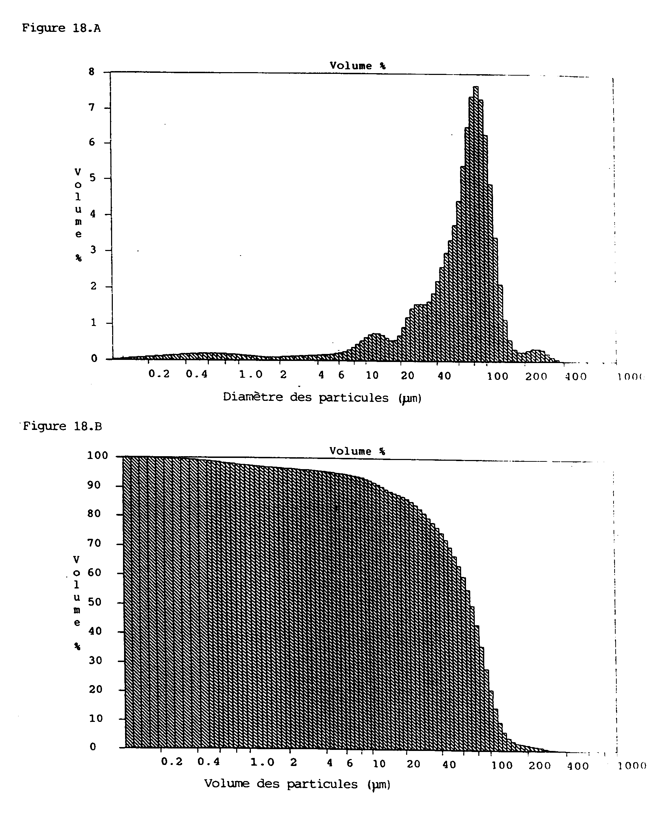

**La figure 18A/21** Volume %

Nom du dossier : 027-S01

Groupe ID : 027

Identification de l'échantillon : TX 066 027

Nombre d'essais : 14

Opérateur : Martine JULIN

Remarques : dispersant Coulter avec anti-mousse

Temps de démarrage : 9 Mai 1990 - 14h34

Durée de l'essai : 61 s

Obscurcissement : 12%

Modèle optique : Fraunhofer

PC : version 1.10 - 13h07 le Vendredi 2 Mars 1990

Statistique de volume (arithmétique) 027 S 01

Calculs de 0,10 $\mu$m à 834,40 $\mu$m

Volume 100,0 %

Moyenne : 69,86 $\mu$m

Médiane : 70,30 $\mu$m

Rapport moyenne/médiane : 0,994

Mode : 86,45 $\mu$m

Limites de confiance à 95% : 61,10 - 78,61 $\mu$m

Déviation standard : 44,67 $\mu$m

Variance : 1995 $\mu$m$^2$

Coefficient de variation : 63,94%

Obliquité : 1,361e = 000 oblique vers la droite

Kurtosis : 5,038e + 000 Leptokurtique

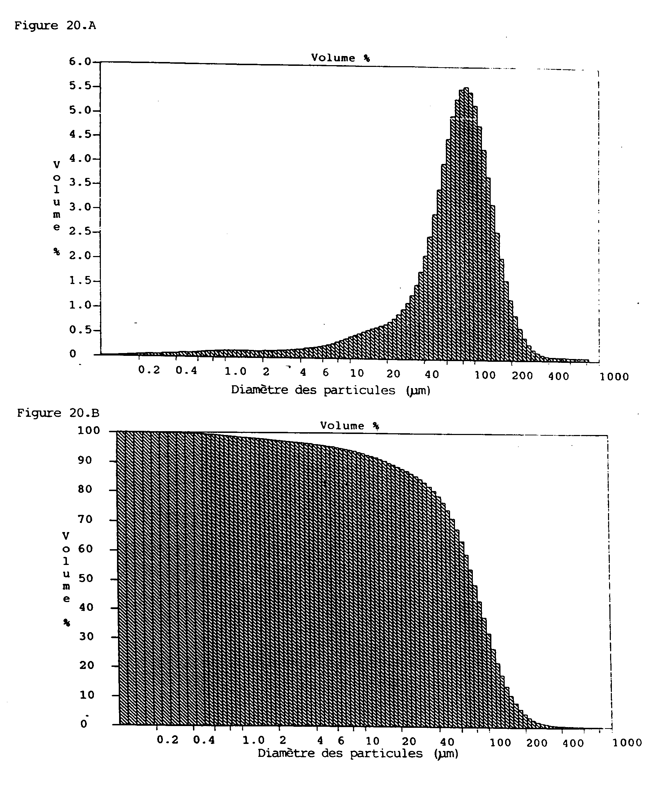

**Les figures 20A/21 et 20B/21**

Statistiques de volume (arithmétique) 035B - S01

Calculs de 0,10 $\mu$m à 834,40 $\mu$m

Volume : 100%

**La figure 21A.1/21**

$t_o$ = 00:07 $t_d$ = 03:36 F∞ = 17,5 $\beta$ = 1,914 RSD = 0,04813

1 - statut de l'essai

2 - élimination

3 - replacement

4 - mode graphique

5 - rapport d'impression

6 - isolation

7 - adaptation au mode

Système de dissolution des comprimés Philips PU 8620 V1.0 date 04/10/91 temps

**La figure 21A.2/21**

$t_o$ = 00:00 $t_d$ = 254:48 F∞ = 461,4 $\beta$ = 0,148 RSD = 0,30028

1 - statut de l'essai

2 - élimination

3 - replacement

4 - mode graphique

5 - rapport d'impression

6 - isolation

7 - adaptation au mode

Système de dissolution des comprimés Philips PU 8629 V1,0 date 14/10/91 temps

**La figure 21B.1/21**

G XVIII 190 meilleure adaptation selon Weibull G XVIII 182

$$t_o = 00{:}07 \quad t_d = 00{:}47 \quad F\infty = 26{,}4 \quad \beta = 1{,}096 \quad RSD = 0{,}03932$$

Système de dissolution des comprimés Philips PU 8620 V1.0 date 04/10/91 temps

**La figure 21B.2/21**

G XVIII 198 meilleure adaptation Weibull TX 47 066 Lot 04

$$t_o = 00{:}07 \quad t_d = 00{:}43 \quad F\infty = 24{,}0 \quad \beta = 1{,}015 \quad RSD = 0{,}04808$$

**Revendications**

1. Un procédé de cristallisation qui permet sans opération mécanique d'obtenir une classe granulométrique homogène que l'on peut déterminer à l'avance, caractérisé en ce que le produit que l'on cherche à faire cristalliser est au préalable, dissout dans un mélange ternaire composé d'un solvant lipophile, d'un solvant hydrophile et d'un agent tensio-actif, à une température proche de l'ébullition du mélange de solvants et qu'on laisse revenir ce mélange à une température où la cristallisation s'amorée puis sépare les cristaux formés.

2. Un procédé selon la revendication 1° dans lequel le produit est un principe actif pharmaceutique, à structure stéroidienne.

3. Un procédé selon l'une des revendications 1 et 2° dans lequel le principe actif est un dérivé de l'estrane.

4. Un procédé selon l'une des revendications 1 et 2° dans lequel le principe actif est un dérivé de l'androstane.

5. Un procédé selon l'une des revendications 1 et 2° dans lequel le principe actif est un dérivé du pregnane.

6. Un procédé selon l'une des revendications 1 et 2° dans lequel le principe actif est un dérivé du 19-nor pregnane.

7. Un procédé selon la revendication 1 ou 2° dans lequel le principe actif est un dérivé du cholestane.

8. Un procédé selon la revendication 1 ou 2° dans lequel le principe actif est un dérivé 21-hydroxy $\Delta 4$-pregnénique.

9. Un procédé selon la revendication 1 ou 3° dans lequel le dérivé de l'estrane est choisi dans le groupe constitué par l'estradiol, l'estrone, l'estriol, la 19-nor Testostérone, les mono-ethers en 3 de ces composés, les diéthers en 3,17 de ces composés et les esters de ces composés.

10. Un procédé selon la revendication 1 ou 4° dans lequel le dérivé de l'androstane est choisi dans le groupe constitué par la testostérone, les éthers de Testostérone, les esters de Testostérone, les dérivés substitués de Testostérone en position 4,6,7 ou 16 par un halogène ou un alcoyle inférieur et le dérivé 17α-éthynyl 17β-acétoxy 5α-endrostène-2.

**11.** Un procédé selon la revendication 1 ou 5° dans lequel le dérivé du pregnane est un stéroide choisi dans le groupe formé de la progestérone, les ethers énoliques de progestérone, les enamines cycliques ou linéaires de progestérone, les 17α hydroxy progestérones, les esters de 17α-hydroxy progestérone, les progestérones substituées par un alcoyle, un trifluorométhyle ou un halogène en position 1, en position 6, en position 7 et/ou en position 16 et les éthers ou les esters de ce dernier composé.

**12.** Un procédé selon la revendication 1 ou 6 dans lequel le dérivé du 19-nor pregnane est un stéroide choisi dans le groupe formé de la 17α-hydroxy 19-nor progestérone, les éthers en 17 de 17α-hydroxy 19-nor progestérone, les esters en 17 de 17α-hydroxy 19-nor progestérone, la 6-méthyl 17α-hydroxy 19-nor progestérone, les éthers en 17 de 6-méthyl 17α-hydroxy 19-nor progestérone, les esters en 17 de 6-méthyl 17α-hydroxy 19-nor progestérone, le 6-méthyl 3,20-dioxo 17α-hydroxy 19-nor pregna 4,6-diène et les esters en 17 de 6-méthyl 17α-hydroxy 3,20-dioxo 19-nor pregna 4,6-diène et les dérivés 17α et 21 méthyl ou éthyl de procédé 3,20-dioxo 19-nor pregna 4,6-diène.

**13.** Un procédé selon la revendication 1 ou 8° dans lequel le dérivé 21-hydroxy Δ4-pregnénique est un corticostéroide choisi dans le groupe constitué par la cortisone, la prednitine, la dexamethasone, la betamethasone, la trianicinolone, le Medrol, le Cortivazol, leurs esters en 17, leurs diesters en 17 et 21 et leurs esters en 21.

**14.** Un procédé selon la revendication 1° dans lequel le solvant lipophile est choisi dans le groupe constitué par les alcanols, les cétones, les esters d'alcoyle et les éthers cycliques dans lesquels le solvant hydrophile peut être miscible en proportion allant jusqu'à 12%.

**15.** Un procédé selon la revendication 14° dans lequel le solvant hydrophile est choisi de façon à se mêler au solvant lipophile pour assurer une solution homogène.

**16.** Un procédé selon la revendication 14° dans lequel le solvant hydrophile est un mélange aqueux formé d'un ou plusieurs solvants oxygénés.

**17.** Un procédé selon la revendication 14° dans lequel le solvant hydrophile est choisi parmi l'eau et/ou les solvants polaires et/ou les cycloalcoyl carboxylates d'alcoyle inférieur.

**18.** Un procédé selon la revendication 1° dans lequel l'agent tensio-actif est un agent tensio-actif non ionique.

**19.** Un procédé selon la revendication 18° dans lequel l'agent tensio-actif non ionique est choisi parmi les esters poly-oxyethyléniques de sorbitanne et d'acide gras ayant au moins 8 atomes de carbone, les éthers poly-oxyéthyléniques d'alcool gras ayant au moins 8 atomes de carbone et les esters poly-oxyethyléniques d'acide stéarique, les copolymères d'oxyde d'éthylène et d'oxyde de propylène.

**20.** Un procédé selon l'une des revendications 18 et 19° dans lequel l'agent tensio-actif non ionique est soluble dans le solvant lipophile ou dans le solvant hydrophile et nécessairement dans le mélange des deux solvants à la fois.

**21.** Un procédé selon la revendication 1° dans lequel le mélange ternaire solvant lipophile, solvant hydrophile et d'agent tensio-actif est réalisé en une ou plusieurs opérations.

**22.** Un procédé selon la revendication 1° dans lequel la concentration en agent tensio-actif dans le mélange ternaire est comprise entre 0,01 et 10%.

**23.** Un procédé selon la revendication 22 dans lequel la teneur en agent tensio-actif dans le solvant ternaire est comptis de préférence entre 0,05 et 5%.

**24.** Un procédé selon la revendication 1° dans lequel le mélange ternaire contenant le principe actif a recristalliser, est amené à une température la plus proche possible de l'ébullition de manière à assurer une concentration la plus élevée possible de la substance à recristalliser et de manière à pouvoir abaisser le plus possible la température de recristallisation de la substance.

25. Des compositions pharmaceutiques renfermant à titre de principe actif un dérivé à structure stéroidienne obtenu selon le procédé de la revendication 1° et de la revendication 2° en mélange ou en association avec un excipient ou un véhicule inerte, non toxique, pharmaceutiquement-acceptable.

26. Des compositions pharmaceutiques selon la revendication 25° dans lesquelles le principe actif est un dérivé du 19-nor pregnane choisi dans le groupe formé par le Nomegestrol et ses esters en 17 obtenu par le procédé de la revendication 6°.

27. Des compositions pharmaceutiques selon la revendication 25° dans lesquelles le principe actif est un dérivé de l'estrane choisi dans le groupe formé de l'estradiol, des éthers en 17 de l'estradiol et des diéthers en 3 et 17 de l'estradiol obtenu par le procédé de la revendication 3°.

28. Des compositions pharmaceutiques selon la revendication 25° dans lesquelles le principe actif est un dérivé de l'androstane choisi dans le groupe formé par la Testostérone, la 19-nor Testostérone, les esters d'acides gras de Testostérone et les esters d'acide gras de 19-nor Testostérone obtenu par le procédé de la revendication 4°.

29. Des compositions pharmaceutiques selon la revendication 25° dans lesquelles le principe actif est un dérivé 21-hydroxy pregnénique choisi dans le groupe formé de la cortisone, la Prednisone, la Dexamethasone, la Betamétasone, le Mediol ainsi que leurs esters en 17 et/ou en 21 obtenus selon le procédé de la revendication 8°.

30. Les micro-cristaux de produit selon la revendication 2° chaque fois qu'ils sont obtenus selon le procédé de la revendication 1°.

**Claims**

1. A process for crystallising a product which allows, whithout mechanical procedure, to obtain a homogen granulometric class which may be prepared beforehand, wherein the product which is desired to be crystallised, is previously dissolved in a ternary mixture made of a lipophilic solvent, a hydrophilic solvent and a surface-active agent, at a temperature close to the boiling point of the mixture of solvents, allowing the mixture of solvents to revert to a temperature where the crystallisation starts, then the thus-formed crystals are recovered.

2. A process according to claim 1 wherein the product to be crystallised is a pharmaceutically active ingredient of steroidal structure.

3. A process according to any of claims 1 and 2 wherein the active ingredient is an estrane derivative.

4. A process according to any of claims 1 and 2 wherein the active ingredient is an androstane derivative.

5. A process according to any of claims 1 and 2 wherein the active ingredient is a pregnane derivative.

6. A process according to any of claims 1 and 2 wherein the active ingredient is a 19-nor pregnane derivative.

7. A process accordint to any of claims 1 and 2 wherein the active ingredient is a derivative of cholestane.

8. A process according to any of claims 1 and 2 wherein the active ingredient is a derivative of 21-hydroxy Δ4-pregnene.

9. A process according to claim 1 or claim 3 wherein the estrane derivative is selected from the group consisting of estradiol, estrone, estriol, 19-nor Testosterone, the 3-mono ethers of the same, the 3,17-diethers of these compounds and the esters of these compounds.

10. A process according to claim 1 or 4 wherein the derivative of androstane is selected from the group consisting of Testosterone, ethers of Testosterone, esters of Testosterone, the Testosterones substituted in position 4,6,7 or 16 with a halogen or a lower alkyl and 17α-ethynyl 17β-acetoxy 5α-androst 2-en.

11. A process according to claim 1 or claims 5 wherein the pregnane derivative is a steroidal compound selected from the group consisting of progesterone, enolic ethers of progesterones, cyclic or linear enamines of progesterone, esters of 17α-hydroxy progesterones, esters of 17α-hydroxy progesterone, progesterones substituted by an alkyl, a trifluoromethyl or a halogen in position 1,6,7 and/or 16, and the ethers or esters thereof.

12. A process according to claim 1 or claim 6 vherein the derivative of 19-nor pregnane is a steroidal derivative selected from the group consisting of 17α-hydroxy 19-nor progesterone, the ethers in 17 of 17α-hydroxy 19-nor progesterone, 6-methyl 17α-hydroxy 19-nor progesterone, ethers in position 17 of 6-methyl 17α-hydroxy 19-nor progesterone, esters in position 17 of 6-methyl 17α-hydroxy 19-nor progesterone, 6-methyl 3,20-dioxo 17α-hydroxy 19-nor pregna 4,6-den and the esters in postion 17 of 6-methyl 17α-hydroxy19-nor pregna 4,6-diene and the 17α- and 21-methyl or ethyl analogs of 6-methyl 3,20-dioxo 19-nor pregna 4,6-dien.

13. A process according to claim 1 wherein the 21-hydroxy Δ4-pregnenic derivative is a corticosteroid selected from the group consisting of Cortisone, Prednisone, Dexamethasone, Betametasone, Triamcinolone, Medrol, Cortivazol, their esters in position 17, their diesters in position 17 and 21 and their esters in position 21.

14. A process according to claim 1 wherein the lipophilic solvent is a solvent selected from the group consisting of alkanols, ketones, alkyl esters and cyclic ethers in which the hydrophilic solvent has to be miscible in an amount up to 12%.

15. A process according to claim 14 wherein the hydrophilic solvent is selected in such a manner to be miscible with the lipophilic solvent in order to insure a homogeneous solution.

16. A process according to claim 14 wherein the hydrophilic solvent is an mixture made of one or several oxygenated solvents, in water.

17. A process according to claim 14 wherein the hydrophilic solvent is selected from the group consisting of water and/or polar solvents and/or lower alkyl esters of cycloalkylcarboxylates.

18. A process according to claim 1 wherein the surfactive agent is an anionic surfactive agent..

19. A process according to claim 1 wherein the anionic surfactive agent is selected from the group consisting of the polyoxyethylenic esters of sorbitan and fatty acids, having at least 8 carbon atoms, the polyoxyethylenic esters of stearic acid and the copolymers of ethylene oxyde and propylene oxyde.

20. A process according to any of claims 18 and 19 wherein the non-ionic surfactive agent is soluble in the lipophilic solvent or in the hydrophilic solvent and necessarily at the same time in the mixture of both solvents.

21. A process according to claim 1 wherein the ternary mixture, made of a lipophilic solvent, a hydrophilic solvent and a surfactive agent, is realized in one or several steps.

22. A process according to claim 1 wherein the concentration in surfactive agent in the ternary mixture lies between 0.01 and 10%

23. A process according to claim 22 wherein the concentration in surfactive agent in the ternary mixture preferably lies between 0.05 and 5%.

24. A process according to claim 1 wherein the ternary mixture containing the active ingredient to be crystallized, is heated to a temperature as close as possible of the boiling point, in order to insure the highest possible concentration of the compound to be crystallized and to decrease as much as possible the temperature of crystallisation of the said compound.

25. Pharmaceutical compositions containing as active ingredient a compound with a steroidal structure obtained by the process of claim 1 or claim 2, in admixture or conjunction with an inert non-toxic, pharmaceutically-acceptable carrier or vehicle.

26. Pharmaceutical compositions according to claim 25 wherein the active ingredient is a 19-nor pregnane derivative selected from the group consisting of Nomegestrol and the esters in position 17 thereof, whenever obtained by the process of claim 6.

27. Pharmaceutical compositions according to claim 25 wherein the active ingredient is an estrane derivative selected from the group consisting of estradiol, ethers of estradiol in position 17, diethers of estradiol in position 3 and 17, whenever obtained by the process of claim 3.

28. Pharmaceutical compositions according to claim 25 wherein the active ingredient is an androstane derivative selected from the group consisting of Testosterone, 19-nor Testosterone, fatty acids esters of Testosterone and the fatty acids esters of 19-nor Testosterone, whenever obtained by the process of claim 4.

29. Pharmaceutical compositions according to claim 25 wherein the active ingredient is a 21-hydroxy pregnenic derivative selected from the group consisting of Cortisone, Prednisone, Dexamethasone, Betamethasone, Medrol and the esters in position 17 and/or in position 21 thereof, whenever obtained by the process of claim 8.

30. The microcrystallized products according to claim 2 whenever obtained by the process of claim 1.

**Patentansprüche**

1. Verfahren zur Kristallisierung eines Produktes, das ohne mekanische Vorgang eine Klasse von homogenen Korngrösse erlaubt, die vorbestimmt verden kann, dadurch gekennzeichnet, dass sich das Produkt das Kristallisieren erwarten lässt, zuvor in einer ternären Mischung der aus einem lipophilen Lösungsmittel, einem hydrophilen losungsmittel und aus einem Tenside besteht, bei einem Temperatur in der Nähe des Siedens des Lösungsmittels aufgelöst ist, dass man diese Mischung bei einer Temperatur vobei die Kristallisation anfängt, abkühlen lässt und dass man die so gebildete Kristalle abtrennt.

2. Verfahren nach Anspruch 1, worin das Produkt eine pharmazeutische Wirkstoff mit steroidischen Struktur ist.

3. Verfahren nach einem der Ansprüche 1 und 2, worin der Wirkstoff eine Oestran Verbindung ist.

4. Verfahren nach einem der Ansprüche 1 und 2, worin der Wirkstoff eine Androstan Verbindung ist.

5. Verfahren nach einem der Ansprüche 1 und 2, worin der Wirkstoff eine Pregnan Verbindung ist.

6. Verfahren nach einem der Ansprüche 1 und 2, worin der Wirkstoff eine 19-Nor Pregnan Verbindung ist.

7. Verfahren nach einem der Ansprüche 1 oder 2, worin der Wirkstoff eine Cholestan Verbindung ist.

8. Verfahren nach einem der Ansprüche 1 oder 2, worin der Wirkstoff eine 21-Hydroxy $\Delta$-4 pregnen Verbindung ist.

9. Verfahren nach Anspruch 1 oder 3, worin die oestran Verbindung aus der Gruppe aus Oestradiol, Oestron, Oestriol, 19-nor Testosteron, den Monoethern in 3-Stellung dieser Verbindungen, den Diethern in 3 und 17-Stellungen dieser Verbindungen und den Estern dieser Verbindungen bestehend, gewählt ist.

10. Verfahren nach Anspruch 1 oder 4, worin die androstan Verbindung aus der Gruppe aus Testosteron, den Ethern von Testosteron, den Estern von Testosteron, den mit einem Halogen oder einem niedrige Alkyl in der Stellung 4,6,7 oder 16 substituierten Derivaten von Testosteron und der 17α-Ethynyl 17β-acetoxy 5α Δ2-Androsten Verbindung bestehend, ausgewählt ist.

11. Verfahren nach Anspruch 1 oder 5, worin die pregnan Verbindung aus die Steroiden aus der Gruppe des Progesterons, der enolischen Ethern des Progesterons, der zyklischen oder geradkettige Enaminen des Progesterons, der 17α-Hydroxy progesteronen, der Estern von 17α-Hydroxy progesteron, der mit einem Alkyl, einem Trifluormethyl, oder einem Halogen substituierten in der Stellung 1, in der Stellung 6, in der Stellung 7 und/oder in der Stellung 16 Progesteronen sowie den Ethern und den Estern der letzte Verbindung bestehend, ausgewählt ist.

12. Verfahren nach Anspruch 1 oder 6, worin die 19-nor pregnane Verbindung aus die Steroiden aus der Gruppe von 17α-Hydroxy 19-nor progesteron, der Ethern in 17-Stellung von 17α-Hydroxy 19-nor progesteron, der Estern in 17-Stellung von 17α-Hydroxy 19-nor progesteron, 6-Methyl 17α-Hydroxy 19-nor progesteron, der Ethern in 17-Stellung von 6-Methyl 17α-Hydroxy 19-nor progesteron, der Estern in 17-Stellung von 6-Methyl 17α-Hydroxy 19-nor progesteron, vom 6-Methyl 3,20-dioxo 17α-Hydroxy 19-nor Pregna 4,6-dien, und der Estern in 17-Stellung von 6-Methyl 17α-Hydroxy 3,20-Dioxo 19-nor pregna 4,6-dien sowie die 17α- und 21-Methyl oder äthyl Derivaten von 3,20-Dioxo 19-nor pregna 4,6-dien bestehend, ausgewählt ist.

13. Verfahren nach Anspruch 1 oder 8, worin das 21-Hydroxy Δ4-pregnen Derivat ein Corticosteroide aus der Gruppe aus Cortison, Prednison, Dexamethason, Betamethason, Triamicinolon, Medrol, Cortivazol, ihre Estern in 17-Stellung, ihre Diestern in 17- und 21-Stellungen, und ihre Estern in 21-Stellung, ausgewählt ist.

14. Verfahren nach Anspruch 1, worin das lipophilisches Lösungsmittel aus der Gruppe aus Alkanolen, Ketonen, Alkyl Estern und cyclischen Ethern bestehend, ausgewählt ist, bei welchen das hydrophilisches Lösungsmittel vermischbar in einer Anteil bis zu 12 % werden kann.

15. Verfahren nach Anspruch 14, worin das hydrophilisches Lösungsmittel so gewählt ist dass es sich mit dem lipophilischen Lösungsmittel versetzt, um eine gleichmässige Lösung gewährzuleisten.

16. Verfahren nach Anspruch 14, worin das hydrophilisches Lösungsmittel eine wasserige Mischung aus einer oder mehrere sauerstoffhaltige Lösungsmittel ist.

17. Verfahren nach Anspruch 14, worin das hydrophilisches Lösungsmittel aus Wasser und/oder polaren Lösungsmitteln und/oder niedrige Alkyl estern von Cycloalkylcarbonsäure ausgewählt ist.

18. Verfahren nach Anspruch 1, worin das Tensid, eine non-ionisches tensidige Wirkstoff ist.

19. Verfahren nach Anspruch 18, worin das nonionisches Tenside aus die Gruppe von polyoxythylenischen Esters von Sorbitan und Fettsäure mit mindenstens 8 Kohlenstoffatomen, die polyoxyethylenische Ethern von Fettalkoholen mit mindenstens 8 Kohlenstoff Atomen und die oxyethylenischen Polyestern von Stearsäure, die Copolymeren von Ethylene Oxyde und Propylene Oxyde ausgewählt ist.

20. Verfahren nach einem der Ansprüche 18 und 19, worin das nonionisches Tensid in dem lipophilischen Lösungsmittel oder in dem hydrophilischen Lösungsmittel und erforderfalls in der Mischung der beide Lösungsmittel, löslich ist.

21. Verfahren nach Anspruch 1, worin die ternäre Mischung aus lipophilisches Lösungsmittel, hydrophilisches Lösungsmitttel und Tenside in eine oder mehrere Arbeitsgänge erfolgt wird.

22. Verfahren nach Anspruch 1, worin das Gehalt an Tensid in der ternäre Mischung, zwischen 0,01 und 10 % beträgt.

23. Verfahren nach Anspruch 22, worin das Gehalt an Tensid, in dem ternären Lösungsmittel, vorzugsweise zwischen 0,05 und 5 % beträgt.

24. Verfahren nach Anspruch 1, worin die ternäre Mischung, die eine zu umkristallisierende Wirkstoff enthält, auf eine möglischst nähe Temperatur von Sieden zugeführt wird, um möglischst hohe Konzentration von der zu umkristallizierenden Substanz zu gewährleisten und um möglichste niedrige die Temperatur von Umkristallisieren der Substanz, zu sinken.

25. Pharmazeutische Zubereitungen die als Wirstoff eine mit steroidischen Struktur nach dem Verfahren nach Anspruch 1 und Anspruch 2 erhaltene Derivat, in Abmischung oder Zusammenhang mit einem inerten, ungiftigen, pharmazeutische-verträglischen Träger oder Vehikel, enthält.

26. Pharmazeutische Zubereitungen nach Anspruch 25, worin der Wirkstoff ein 19-Nor pregnan Derivat aus der Gruppe der nach dem Verfahren von Anspruch 6 gewonnene Nomegestrol und seine in der Stellung 17 Estern, ist.

27. Pharmazeutische Zubereitungen nach Anspruch 25, worin der Wirkstoff eine nach dem Verfahren des Anspruch 3 gewonnene aus der Gruppe von Oestradiol, die in 17 Stellung Estern von Oestradiol, die in Stellungen-3 und -17 Diestern von Oestradiol ausgewählt, Oestran Derivat, ist.

28. Pharmazeutische Zubereitungen nach Anspruch 25, worin der Wirkstoff ein Derivat des Testosterons aus der Gruppe von Testosteron, 19-nor Testosteron, Testosterons Fettsäure Estern und 19-nor Testosterons Fettsäure Estern gebildet sind, ist, der nach dem Verfahren des Anspruches 4 hergestellt ist.

29. Pharmazeutische Zubereitungen nach Anspruch 25, worin der Wirkstoff ein 21-Hydroxypregnenische Derivat der aus der Gruppe von Cortison, Prednison, Dexamethason, Betamethason, Medrol gebildet ist, ist sowie ihre in 17- und/oder 21 Stellungen Estern, der nach dem Verfahren des Anspruches 8 hergestellt ist.

30. Mikrokristallen von Produkt nach Anspruch 2, jedemal sie nach dem Verfahren gemäss Anspruch 1 hergestellt sind.

Figure 1.A

Figure 1.B

Figure 2.A

Figure 2.B

Figure 3.A

Figure 3.B

Figure 4

Figure 5.A

Figure 5.B

Figure 6

Figure 7

Volume %

Diamètre des particules (µm)

Figure 8.A

Figure 8.B

Figure 9.A

Figure 9.B

Figure 10

Figure 11

Figure 12

Figure 13 A/1

Figure 13 A/2

45

Figure 14/A

Figure 14/B

Figure 15 A/1

Figure 15 A/2

**Figure 16**

Figure 17.A

Figure 17.B

Figure 18.A

Figure 18.B

Figure 19.A

Figure 19.B

Figure 20.A

Volume %

Figure 20.B

Volume %

Figure 21 A/1

100.0
80.0
60.0
40.0
20.0
0.0

00:00    01:24    02:48    04:12    05:36    07:00

1    2    3    4    5    6

Figure 21 A/2

100.0
80.0
60.0
40.0
20.0
0.0

00:00    01:24    02:48    04:12    05:36    07:00

1    2    3    4    5    6

53

Figure 21 B/1

Figure 21 B/2